(19) **Europäisches Patentamt**

**European Patent Office**

**Office européen des brevets**

(11) **EP 1 129 661 B1**

(12) **EUROPÄISCHE PATENTSCHRIFT**

(45) Veröffentlichungstag und Bekanntmachung des Hinweises auf die Patenterteilung:
**12.10.2005 Patentblatt 2005/41**

(51) Int Cl.⁷: **A61B 5/0452**, A61B 5/0456

(21) Anmeldenummer: **01250043.5**

(22) Anmeldetag: **07.02.2001**

(54) **Vorrichtung zur Verarbeitung von Körpersignalen**

Device for processing body signals

Dispositif de traitement de signaux émis par un corps vivant

(84) Benannte Vertragsstaaten:
**CH DE FR GB IT LI NL**

(30) Priorität: **18.02.2000 DE 10008792**

(43) Veröffentlichungstag der Anmeldung:
**05.09.2001 Patentblatt 2001/36**

(73) Patentinhaber: **BIOTRONIK Mess- und Therapiegeräte GmbH & Co Ingenieurbüro Berlin**
**12359 Berlin (DE)**

(72) Erfinder: **Schomburg, Richard A.**
**Hillsboro, Oregon 97123-9048 (US)**

(74) Vertreter: **Eisenführ, Speiser & Partner**
**Patentanwälte Rechtsanwälte**
**Spreepalais am Dom**
**Anna-Louisa-Karsch-Strasse 2**
**10178 Berlin (DE)**

(56) Entgegenhaltungen:
**EP-A- 0 487 429      DE-C- 19 752 094**

## Beschreibung

Die Erfindung betrifft eine Vorrichtung zur Verarbeitung von Körpersignalen, welche einen Sensor zur Aufnahme von insbesondere elektrischen Signalen eines lebenden Körpers umfaßt, sowie Mittel zur Aufbereitung aufgenommener Signale für die weitere Verarbeitung und wenigstens einen ersten Speicher für ein aufgenommenes Meßsignal oder Signalabschnitte davon.

Es ist häufig ein Anliegen in beispielsweise elektrisch aufgenommenen Signalen bestimmte Merkmale zu erkennen. Zum Beispiel kann es interessant sein in einem Elektrokardiogramm T-Wellen oder QRS-Komplexe zu erkennen und den Zeitpunkt ihres Auftretens möglichst genau zu bestimmen. Zur Analyse solcher Signale ist es wünschenswert bestimmte Ereignisse oder Signalmerkmale in zeitlicher Hinsicht zu lokalisieren.

Vor diesem Hintergrund betrifft die vorliegende Erfindung insbesondere die Aufnahme intrakardial aufgenommener Signale insbesondere in einem implantierten Gerät. Aus Gründen des Energiebedarfes und des begrenzten Raumes stehen in einem implantierten Gerät nur begrenzte Ressourcen für die Signalverarbeitung und -analyse zur Verfügung.

Es sind bereits verschiedene Vorrichtungen und Verfahren zur Merkmalserkennung von Herzsignalen und zur Signalanalyse bekannt. In dem Europäischen Patent 0 487 429 ist beispielsweise eine Vorrichtung offenbart, in der eine Folge von Werten solcher Parameter gespeichert wird, die einem aktiven Herzzyklus entsprechen. Die Vorrichtung umfaßt Vergleichseinrichtungen, um die gespeicherten Parameterwerte des aktiven Herzzyklusses mit zuvor gespeicherten Informationen über diese Werte zu vergleichen und im Falle eines positiven Vergleichsergebnisses ein Signal auszulösen. Auch die nicht vorveröffentlichten deutschen Patentanmeldungen 199 38 376 und 199 63 246 betreffen jeweils Vorrichtungen, in denen ein gemessenes Herzsignal mit zuvor gebildeten und abgespeicherten Vergleichssignalen verglichen wird, um im Falle der DE 199 38 376 Fusionsereignisse bei der Elektrostimulation des Herzens zu erkennen und im Falle der DE 199 63 246 die Kreislaufwirkung von Extrasystolen.

Aus dem US-Patent 5,439,483 ist darüber hinaus die Anwendung einer Wavelet-Transformation zur Klassifizierung von Tachykardien bekannt. In der US 5,778,881 wird eine Wavelet-Transformation zusätzlich mit einer Hidden-Markov-Modellierung kombiniert um P-R-Wellen jeweils als Markov-Zustände mit einer verringerten Anzahl von Wavelet-Koeffizienten detektieren zu können. Weiter wird dort vorgeschlagen, einen für das jeweilige Ergebnis typischen Satz von Wavelet-Koeffizienten bei schnellen Änderungen der Signal-Morphologie automatisch zu aktualisieren, um die Analyse von kurzfristigen Schwankungen der physiologischen Signale beispielsweise aufgrund körperlicher Belastung unabhängig zu machen.

Die deutsche Patentschrift DE 19752094 Cl beschreibt eine Vorrichtung gemäß dem Oberbegriff von Anspruch 1.

Ziel der vorliegenden Erfindung ist es, in physiologischen Signalen wie intrakardialen Elektrokardiogrammen (EKG) und intrakardialen dynamischen Impedanzverläufen (IDZ) das Auftreten bestimmter Merkmale und deren Zeitpunkt, im folgenden auch zeitliche Lokation genannt, auf effiziente Weise zu bestimmen. Dies soll möglichst zuverlässig auch in Gegenwart von Rauschen und Störsignalen erfolgen. Die Genauigkeit der zeitlichen Lokation eines Merkmals soll der Sampling- oder Abtastrate, das heißt dem Zeitraster entsprechen, mit dem die physiologischen Signale aufgenommen werden. Die Merkmalserkennung soll auch bei Veränderung der Signal-Morphologie funktionieren.

Weitere Ziele der Erfindung umfassen verbesserte Merkmalsanalysefunktionen.

Der vorliegenden Erfindung liegt somit die Aufgabe zugrunde, gegenüber dem Stand der Technik zumindest in Einzelbereichen eine bessere oder effizientere Signalanalyse zu ermöglichen und damit die vorgenannten Ziele weitestgehend zu erreichen.

Erfindungsgemäß wird diese Aufgabe durch eine Vorrichtung der eingangs genannten Art erreicht, die mindestens einen zweiten Speicher umfaßt, welcher ein vorgebbares, zeitlich endliches Vergleichssignal enthält sowie Signalvergleichsmittel, die mit dem zweiten und dem ersten Speicher verbunden und zum gleitenden Vergleichen sich zeitlich überlappender Signalabschnitte des Meßsignals im ersten Speicher mit dem in dem zweiten Speicher gespeicherten Vergleichsignals und zur Ausgabe eines die Ähnlichkeit eines jedes verglichenen Signalabschnittes des Meßsignals mit dem Vergleichssignal repräsentierenden Korrelationskoeffizienten ausgebildet sind. Hierbei ist das Integral des Vergleichssignals über die Zeit oder dessen Summe zeitlich diskreter Signalwerte Null. Dadurch wird das von den Korrelationskoeffizienten gebildete Korrelationssignal ein Signal, daß einen Mittelwert von etwa Null besitzt. Dies vereinfacht die nachfolgende Signalanalyse sehr.

Das Meßsignal ist hierbei vorzugsweise ein intrakardiales Elektrogramm (EKG) oder ein intrakardial aufgenommener dynamischer Impedanzverlauf (IDZ). Werden diese Signale nicht kontinuierlich aufgenommen, sondern mit einer Abtastrate zeitlich diskret, befindet sich im ersten Speicher eine Folge zeitlich diskreter Meßwerte, die das Meßsignal repräsentieren. Auch das Vegleichssignal wird im zweiten Speicher als endliche Folge zeitlich diskreter Werte abgelegt. In diesem Falle ist statt des Integrals des Vergleichssignals über die Zeit vorzugsweise dessen Summe aller diskreten Signalwerte Null. Dies entspricht den Anforderungen, die an Wavelets gestellt werden. Daher kann das Vergleichssignal auch als Vergleichswavelet bezeichnet werden.

Ein entscheidender Unterschied zur Wavelettransformation besteht darin, daß die Vorrichtung nicht wie die Wavelettransformation ein zweidimensionales Ergebnis liefert, da das Vergleichsmuster nicht für die Untersuchung jedes Meßsignalschrittes über einen bestimmten Frequenzbereich zeitlich skaliert wird. Hierdurch wird der erforderliche Rechenaufwand entscheidend verringert

Die zeitliche Auflösung des Meßsignals im ersten Speicher ist vorzugsweise dieselbe wie diejenige des Vergleichssignals im zweiten Speicher. Jeder mit dem Vergleichssignal zu vergleichende Signalabschnitt des Meßsignals entspricht dann hinsichtlich der Zeitdauer und der Anzahl der diskreten Meßwerte dem Vergleichssignal. Die durch den Vergleich der Signale gebildeten Korrelationskoeffizienten bilden ebenfalls eine Folge zeitlich diskreter Werte, die jeweils die Ähnlichkeit genau eines Signalabschnittes im ersten Speicher mit dem Vergleichssignal repräsentieren. Die entsprechenden Signalabschnitte des im ersten Speicher abgelegten Meßsignals überlappen sich dabei vorzugsweise derart, daß sie nur um einen der zeitlichen Auflösung des Meßsignals entsprechenden diskreten Zeitschritt gegeneinander verschoben sind. Durch diesen gleitenden Vergleich wird ein zeitlich diskretes Korrelationssignal erzeugt, welches von den zeitlich aufeinanderfolgenden Korrelationskoeffizienten als Ergebnis des Vergleichs der Signale gebildet wird. Die zeitliche Auflösung des Korrelationssignals ist dann die gleiche wie die des Meßsignals und des Vergleichssignals. Das Korrelationssignal kann aber auch mit niedrigerer zeitlicher Auflösung gebildet werden, wenn der Vergleich nur für jeden zweiten, dritten oder n-Zeitschritt durchgeführt wird. Die für den Vergleich mit dem Vergleichssignal herangezogenen Signalabschnitte des Meßsignals sind dann von Vergleich zu Vergleich um jeweils zwei, drei oder n Zeitschritte gegeneinander verschoben. In der Praxis wird es häufig jedoch von Vorteil sein, das Meß- und das Vergleichssignal mit einer entsprechend niedrigeren, das Zeitraster definierenden Samplingrate aufzunehmen, sofern die niedrigere zeitliche Signalauflösung noch eine sichere Merkmalserkennung und einen zuverlässigen Signalvergleich zuläßt.

Wesentliches Merkmal der Vorrichtung ist der zweite Speicher, der das Vergleichssignal enthält, welches sich je nach zu detektierendem Signalmerkmal vorgeben läßt und insbesondere zur Anpassung an eine sich verändernde Signal-Morphologie durch die Vorrichtung selbst veränderbar ist.

Bevorzugt ist eine Vorrichtung, bei der die Signalvergleichsmittel mit einem Logarithmenspeicher verbunden sind, der Tabellen von Logarithmen für Werte des Meß- und des Vergleichssignals enthält, wobei die Signalvergleichsmittel zum Bilden der Korrelationskoeffizienten derart ausgebildet sind, daß sie eine Multiplikation eines Wertes des Vergleichssignals aus dem zweiten Speicher mit dem entsprechenden Wert des ersten Meßsignals aus dem ersten Speicher derart durchführen, daß zunächst aus dem Logarithmenspeicher die Logarithmen der zu multiplizierenden Werte selbst oder der ihnen jeweils nächstkommenden Werte ausgelesen werden und anschließend die beiden Logarithmen addiert werden.

Eine derartige durch die Verwendung von Logarithmentafeln oder Rechenschiebern an sich bekannte Vorgehensweise zur Multiplikation zweier Werte kann auf effiziente, speichersparende Weise durchgeführt werden.

Eine bevorzugte Vorrichtung weist weiterhin Detektionsmittel auf, die mit den Signalvergleichsmitteln verbunden und zum Detektieren von Maximalwerten und/oder Nulldurchgängen eines von den Korrelationskoeffizienten gebildeten Signals ausgebildet sind.

Vorzugsweise weist die Vorrichtung weiterhin Schwellwertvergleichsmittel auf, die mit den Signalvergleichsmitteln und einem, einen Schwellwert enthaltenen Schwellwertspeicher verbunden und zur Ausgabe eines Erkennungssignals ausgebildet sind, sobald der von den Signalvergleichsmitteln ausgegebene Korrelationskoeffizient den Schwellwert überschreitet. Dabei ist die Vorrichtung vorzugsweise so ausgebildet, daß die Schwellwertvergleichsmittel so ausgebildet sind, daß sie ein Erkennungssignal ausgeben, wenn ein Korrelationskoeffizient für einen ersten Signalabschnitt aus dem ersten Speicher den Schwellwert überschreitet und für einen zweiten, zeitlich nach dem ersten Signalabschnitt aufgenommenen Signalabschnitt den Wert Null erreicht oder unterschreitet. Zusätzlich sind vorzugsweise Lokationsmittel vorgesehen, die mit den Schwellwertvergleichsmitteln und den Detektionsmitteln verbunden und so ausgebildet sind, daß sie demjenigen Signalabschnitt des Meßsignales in dem ersten Speicher ein Lokationssignal zuordnen, für den das von den Korrelationskoeffizienten gebildete Signal ein Maximum innerhalb desjenigen Ausschnitts des von den Korrelationskoeffizienten gebildeten Signals besitzt, für das die Schwellwertvergleichsmittel ein Erkennungssignal ausgeben.

Da jeder Korrelationskoeffizient der Folge von Korrelationskoeffizienten genau einem Signalabschnitt des im ersten Speicher abgelegten Meßsignals zugeordnet ist, kann durch Bestimmung der entprechenden Maxima der Folge der Korrelationskoeffizienten genau die zeitliche Lokation, also der Ort eines Merkmales in dem untersuchten Signal und damit der Zeitpunkt des Auftretens eines Merkmals, bestimmt werden.

Die Vorrichtung weist weiterhin vorzugsweise Schwellwertbildungsmittel auf, die mit dem Schwellwertspeicher und den Lokationsmitteln verbunden und so ausgebildet sind, daß sie einen neuen Schwellwert nach Auftreten eines Lokationssignals derart bilden, daß der dem Lokationssignal zugeordnete Korrelationskoeffizient gewichtet in die Bildung des neuen Schwellwertes eingeht. Dies erlaubt eine ständige Anpassung des Schwellwertes an den tatsächlichen Verlauf den Meßsignals und an Veränderungen von dessen Morphologie.

Ebenfalls bevorzugt ist eine Vorrichtung, die Vergleichssignalbildungsmittel zum Bilden eines neuen Vergleichssignals aufweist, welche mit dem zweiten Speicher verbunden und so ausgebildet sind, daß ein gemessener Signalabschnitt, der einem zu detektierenden Signalmerkmal entspricht, derart zu dem Vergleichssignal transformiert wird, daß sein Integral über die Zeit oder die Summe der zeitdiskreten Signalwerte Null ist, und das so gebildete Vergleichssignal in den zweiten Speicher übertragen wird. Die Vergleichssignalbildungsmittel erlauben somit eine automatische Bildung eines geeigneten Vergleichssignals.

Auch ist eine Vorrichtung bevorzugt, welche Vergleichssignalanpassungsmittel zum Anpassen des Vergleichssignals umfaßt, die mit dem ersten Speicher, dem zweiten Speicher und den Lokationsmitteln verbunden und so ausgebildet sind, daß sie ein neues, angepaßtes Vergleichssignal bilden, wenn die Lokationsmittel ein Lokationssignal ausgeben, wobei das angepaßte Vergleichssignal unter Verwendung desjenigen Meßsignalabschnittes aus dem ersten Speicher gebildet wird, dem das Lokationssignal zugeordnet ist. Dies erlaubt eine ständige Anpassung des Vergleichssignals an die tatsächliche Morphologie des Meßsignals, mit der Folge, daß sich Charakteristika des Meßsignals in dem Vergleichssignal widerspiegeln, so daß zur Analyse des Meßsignals anstelle des Meßsignals auch das Vergleichssignal analysiert werden kann. Außerdem erlaubt diese Anpassung des Vergleichssignals eine sichere, zuverlässige und zeitgenaue Merkmalsdetektion.

Die Vorrichtung zeichnet sich dabei vorzugsweise durch derart ausgebildete Vergleichssignalanpassungsmittel aus, daß das vor dem Anpassen gültige Vergleichssignal zur Bildung des nach dem Anpassen gültigen Vergleichssignals mit einem Gewichtungsfaktor $1-\alpha$ multipliziert in das neuzubildende Vergleichssignal eingeht, während derjenige Signalabschnitt im ersten Speicher, dem das die Anpassung des Vergleichsignals auslösende Lokationssignal zugeordnet ist, mit einem Gewichtungsfaktor $\alpha$ in das nach dem Anpassen gültige Vergleichssignal eingeht, $\alpha$ ist dabei ein Wert zwischen 0 und 1. Das neue Vergleichssignal entspricht dann der Summe der beiden gewichteten Signale, die in die Bildung des neuen Vergleichssignals eingegangen sind.

Außerdem sind die Vergleichssignalbildungsmittel und/oder die Vergleichssignalanpassungsmittel vorzugsweise derart ausgebildet, daß das gebildete bzw. das angepaßte Vergleichssignal derart normiert ist, daß dessen Amplitude der maximalen Amplitude des Meßsignals entspricht. Auf diese Weise werden unerwünschte Effekte infolge der Signalmultiplikation bei der Bildung der Korrelationskoeffizienten vermieden, die die Skala für den Schwellwert quadratisch verzerren würden.

Vorzugsweise weist die Vorrichtung eine Datenbank auf, welche mehrere Vergleichssignale enthält und derart mit dem zweiten Speicher verbunden ist, daß Vergleichssignale von der Datenbank in den zweiten Speicher und umgekehrt übertragbar sind. Auf diese Weise kann die Vorrichtung mit verschiedenen Vergleichssignalen zur Detektion verschiedener Signalmerkmale operieren.

Außerdem weist die Vorrichtung vorzugsweise Analysemittel auf, die zur Analyse der kennzeichnenden Eigenschaften des vorzugsweise angepaßten Vergleichssignals ausgebildet sind. Derartige Analysemittel erlauben die Analyse des Meßsignals indirekt durch Auswertung des an das Meßsignal angepaßten Vergleichssignals.

In einer bevorzugten Variante umfaßt die Vorrichtung Mittel zum Aufnehmen zweier Herzsignale, von denen eins dem linken und das andere dem rechten Ventrikel bzw. Atrium zugeordnet ist, sowie mit diesem Mittel verbundene Mittel zum Bilden eines bimodalen Signals aus den zwei Herzsignalen, dergestalt, daß das bimodale Signal ein Merkmal des ersten Signals vor seiner Überleitung in das jeweils andere Ventrikel oder Atrium und das entsprechende Merkmal nach seiner Überleitung enthält, so daß das Merkmal in einem der Überleitungszeit entsprechenden zeitlichen Abstand einmal in seiner Form vor und einmal in seiner Form nach der Überleitung in dem bimodalen Signal enthalten ist. Außerdem enthält der zweite Speicher dieser Vorrichtung ein bimodales Vergleichssignal, welches an das bimodale Signal anpaßbar ist, so daß nach Anpassung des bimodalen Vergleichssignals an das bimodale Signal die Überleitungszeit durch eine Analyse des Vergleichssignals bestimmbar ist. Diese erlaubt im Kontext der beschriebenen Vorrichtung eine sehr genaue Bestimmung der Überleitungszeit zwischen zwei Herzkammern.

Die Erfindung soll nun anhand eines Ausführungsbeispiels mit Hilfe der Figuren näher erläutert werden. Von den Figuren zeigt:

Fig. 1     ein Blockdiagramm einer erfindungsgemäßen Vorrichtung;

Fig. 2     ein Vergleichssignal in Form eines Wavelets vom Typ "Mexikanerhut";

Fig. 3     ein Vergleichssignal in Form eines asymmetrischen Wavelets;

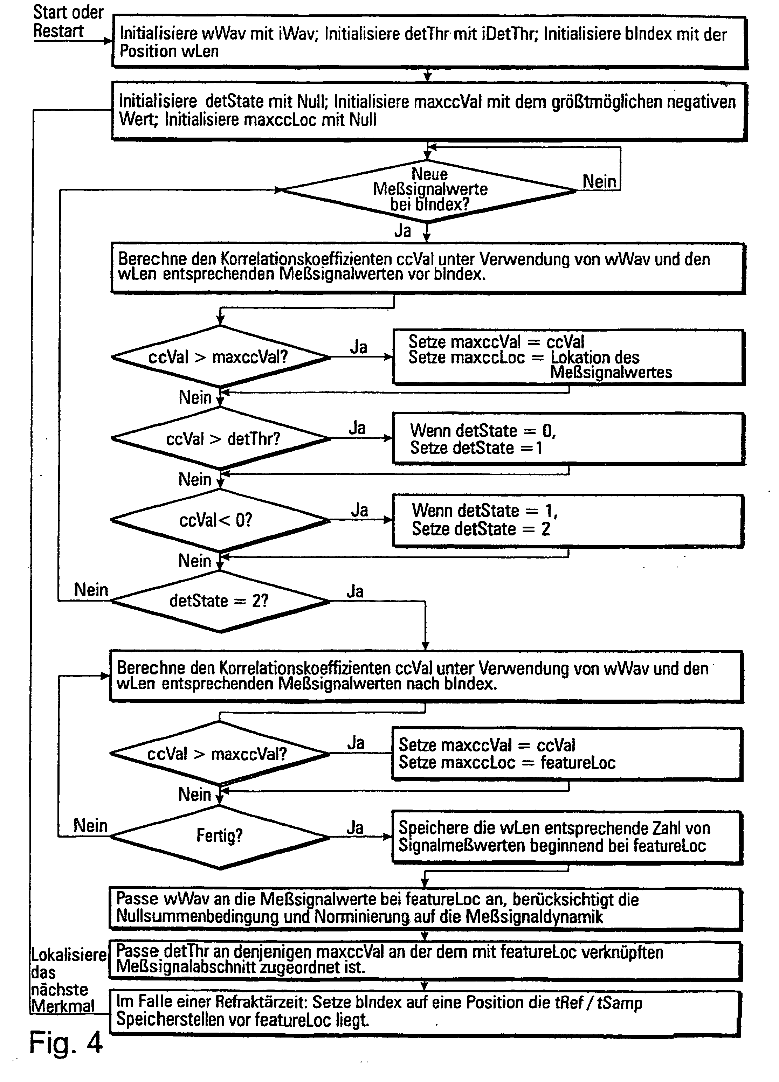

Fig. 4     ein Flußdiagramm eines Detektions-/Lokationsalgorithmusses;

Fig. 5     ein Vergleichssignal mit bimodaler Struktur;

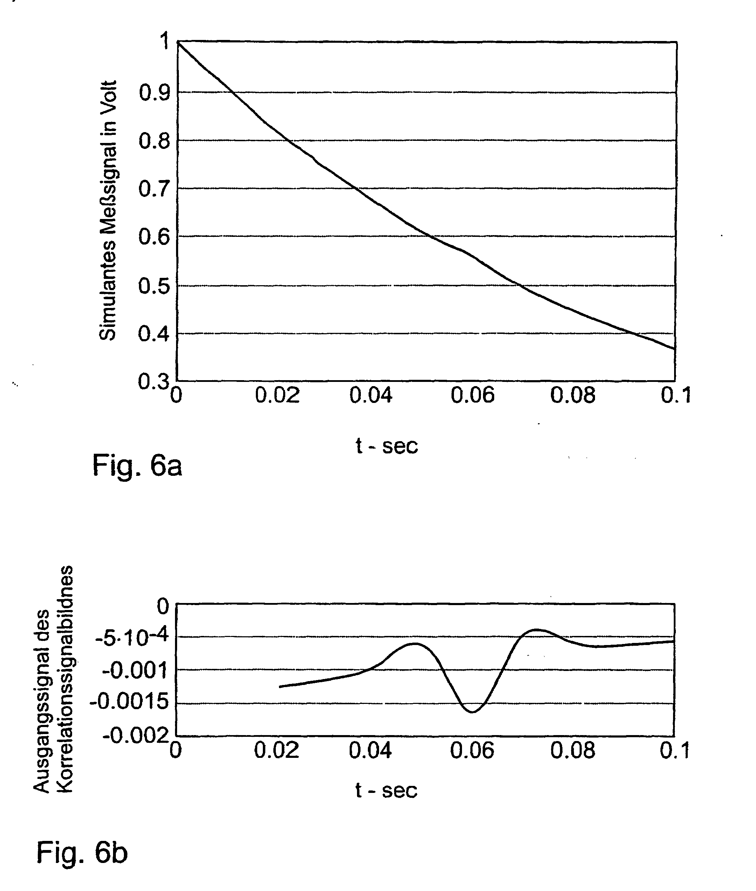

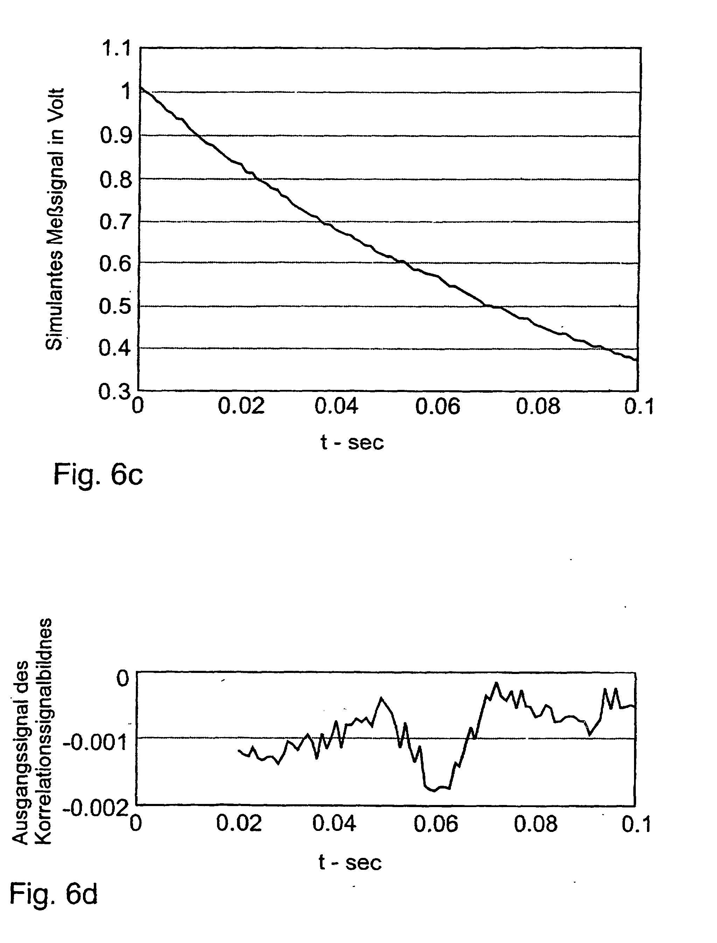

Fig. 6a-d     das Ergebnis der Simulation eines Detektors für eine evozierte Herzreaktion.

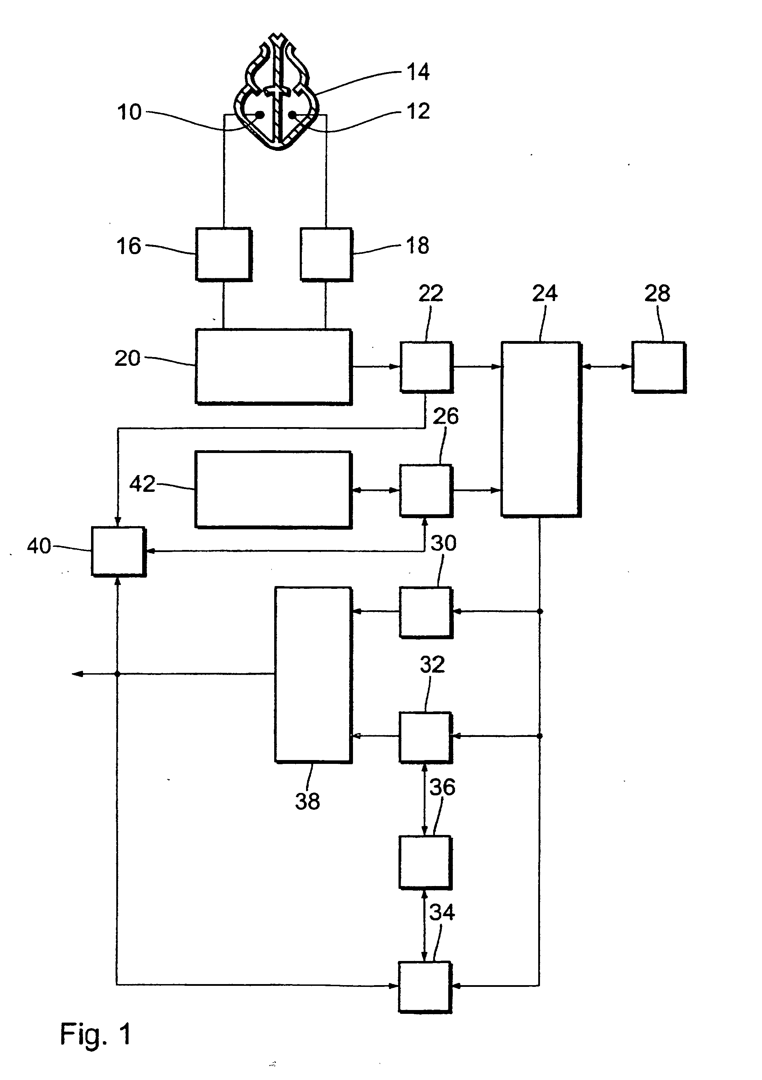

Dem Blockdiagramm in Figur 1 sind zwei Signalaufnehmer 10 und 12 zu entnehmen, die jeweils in der linken und rechten Kammer eines Herzens 14 plaziert sind. Mit den Signalaufnehmern 10 und 12 sind jeweils ein Meßverstärker 16 und 18 verbunden. Über diese gelangt das von den Meßwertaufnehmern 10 und 12 aufgenommene Signal in eine Meßsignalaufbereitungseinheit 20, die mit einem ersten Speicher 22 für das Meßsignal verbunden ist und in diesen wahlweise eine zeitlich diskrete Folge von Meßwerten hineinschreibt, die entweder das aus der linken oder aus der rechten Herzkammer stammende Meßsignal oder ein aus beiden zusammengesetztes bimodales Meßsignal repräsentieren. Der erste Speicher 22 ist mit einem Korrelationssignalbildner 24 verbunden, der seinerseits außerdem mit einem zweiten Speicher 26 für ein Vergleichssignal und einem Logarithmenspeicher 28 verbunden ist. Der Korrelationssignalbildner 24 bildet auf weiter hinten beschriebene Weise ein zeitlich diskretes Korrelationssignal und gibt dieses an einen Detektor 30, eine Erkennungseinheit 32 und einen Schwellwertbildner 34 weiter. Der Detektor 30 ist zum Detektieren lokaler Maxima und der Nulldurchgänge des Korrelationssignals ausgebildet, während die Erkennungseinheit 32 ein Signal ausgibt, sobald das Korrelationssignal einen größeren Wert annimmt, als in einem Schwellwertspeicher 36 abgelegt ist, der mit der Erkennungseinheit 32 verbunden ist. Der Schwellwertspeicher 36 ist außerdem mit dem Schwellwertbildner 34 verbunden. Der Schwellwertbildner 34 enthält einen Speicher für den zuvor beschriebenen Gewichtungsfaktor $\alpha$

Der Detektor 30 und die Erkennungseinheit 32 sind ausgangsseitig jeweils mit einem Lokationsdetektor 38 verbunden, der in zuvor beschriebener Weise ein Lokationssignal ausgibt, wenn das Korrelationssignal ein lokales, über dem Schwellwert liegendes Maximum gefolgt von einem Nulldurchgang aufweist. Das Lokationssignal ist einem definierten Abschnitt des Meßsignals in dem ersten Speicher 22 genau zugeordnet. Der Lokationsdetektor 38 ist ausgangsseitig sowohl mit dem Schwellwertbildner 34 als auch mit einem Vergleichssignalbildner 40 verbunden. Der Vergleichssignalbildner 40 enthält einen Speicher für die Werte der weiter hinten beschriebenen Parameter $\beta$ und $\gamma$ für die Vergleichssignalanpassung.

Auf ein Lokationssignal des Lokationsdetektors 38 hin bildet der Schwellwertbildner auf die andernorts beschriebene Weise aus dem dem Lokationssignal zugeordneten Wert des Korrelationssignals und dem Schwellwertspeicher 36 abgelegten Schwellwerte einen neuen Schwellwert und legt diesen anstelle des alten in dem Schwellwertspeicher 36 ab.

In ähnlicher Weise löst das Lokationssignal des Lokationsdetektors 38 die Bildung eines neuen Vergleichssignals in dem Vergleichssignalbildner 40 aus. Der Vergleichssignalbildner 40 bildet das neue Vergleichssignal, in das das alte Vergleichssignal und derjenige Meßsignalabschnitt, der dem Lokationssignal zugeordnet ist, jeweils gewichtet eingehen. Anschließend wird das alte Vergleichssignal durch das neue in dem zweiten Speicher 26 ersetzt. Der zweite Speicher 26 ist außerdem mit einer Datenbank 42 verbunden, die verschiedene Vergleichssignale für verschiedene zu detektierende Merkmale des Meßsignales enthält.

Die beschriebenen Bestandteile der Vorrichtung können zumindest teilweise durch Softwaremodule und einen Mikroprozessor realisiert sein.

In der Vorrichtung kommen Korrelationsmethoden zur Anwendung, welche teilweise an Anwendungen der Wavelettransformation erinnern, sich von dieser aber in verschiedenen wichtigen Punkten unterscheiden. Dies wird in den folgenden Abschnitten beschrieben.

In einfachen Worten ist die Wavelettransformation eine mathematische Methode, welche auf eine Eingangsfunktion, beispielsweise ein Spannungssignal, angewandt wird, welche die Funktion einer unabhängigen Variablen, beipielsweise der Zeit ist. Für die Wavelettransformation wird ein spezifisches, zeitlich begrenztes Signal, das Wavelet, verwendet, welches eine Funktion derselben unabhängigen Variablen ist, wie die Eingangsfunktion. Die Wavelettransformation liefert ein zweidimensionales Ergebnis, welches das gegenüber der Zeit aufgetragene Frequenzverhalten der Eingangsfunktion widerspiegelt. Dieses Frequenzverhalten wird dadurch beschrieben, daß ein Wavelet in einem interessierenden Parameterbereich in Bezug auf die unabhängige Variable also beispielsweise zeitlich und damit spektral skaliert wird und das so skalierte Ausgangswavelet mit der Eingangsfunktion verglichen wird. Die Frequenzachse des zweidimensionalen Ergebnisses der Wavelettransformation entspricht dabei dem Skalierungsfaktor des Wavelets, wobei die höheren Frequenzen weiter entfernt vom Ursprung eines Koordinatensystems aufgetragen werden. Jeder Punkt der zweidimensionalen Ausgangsfunktion hat den Wert eines Korrelationskoeffizienten, der die Korrelation des Eingangssignals zu dem entsprechenden Zeitpunkt mit demjenigen Wavelet widerspiegelt, das dem entsprechenden Skalierungsfaktor zugeordnet ist. Auf diese Weise ergibt die Wavelettransformation eine zweidimensionale Ausgangsfunktion, die die Ähnlichkeit zwischen einer Eingangsfunktion bzw. einem Eingangssignal und einem jeweils verschieden skalierten Wavelet widerspiegelt, wobei eine Koordinate der zweidimensionalen Ausgangsfunktion der Skalierungsfaktor des Wavelets und die andere Koordinate die unabhängige Variable der Eingangsfunktion, beispielsweise die Zeit ist.

Im Falle kontinuierlicher Eingangssignale oder -funktionen kann die Wavelettransformation durch die folgende Gleichung beschrieben werden:

$$w(s,\tau) = \int f(t)\,^{*}\Psi(st+\tau)\,dt$$

In dieser Funktion ist $\psi$ eine kontinuierliche "Stammwavelet"-Funktion, die von einem Skalierungsparameter s und einem Translationsparameter $\tau$ abhängt. Um ein Wavelet zu sein, muß die Funktion einen Mittelwert von Null haben und mit zunehmenden Werten ihres Argumentes abfallen, um hinsichtlich Zeit und Frequenz lokalisiert zu sein. Die dargestellte Integration muß nur über den Zeitbereich ausgeführt werden, für den die Koeffizienten von $\psi$ ungleich Null sind.

Für diskrete Eingangsfunktionen oder -signale kann die Wavelettransformation durch die folgende Formel ausgedrückt werden:

$$W(s, n\ \tau) = \sum_{k} f(\tau(n+k))\,{*}\,\Psi(s,k)$$

In dieser Formel ist n ein Zähler für einen Abtastwert, $\tau$ das Abtastintervall und k ein Index für den Abtastwertzähler des Wavelets $\psi$ mit dem Skalierungsfaktor s.

Für ein System mit diskreten Eingangsfunktionen kann die Korrelationsfunktion zwischen einem Eingangssignal und einem vorgegebenen (Vergleichssignal oder auch Korrelationssignal oder Korrelatorwavelet), $\psi$, ausgedrückt werden als :

$$C(n\ \tau) = \sum_{k} f(\tau(n+k))\,{*}\,\Psi(k)$$

Darin ist n die Nummer des Abtastwertes, $\tau$ das Abtastintervall und k ein Index für die Abtastwertnummer des Wavelets. Durch Vergleich der beiden zuletzt aufgeführten Gleichungen wird offenbar, daß die Korrelationsfunktion als ein besonderer Fall der Wavelettransformation angesehen werden kann, für die der Skalierungsfaktor s konstant ist.

Die Vorrichtung beruht auf Methoden, die die Berechnung und Interpretation von Korrelationsfunktionen einschließen, für die ein oder mehrere Vergleichssignale oder Korrelator-Wavelets nicht konstant sind, sondern angepaßt werden, damit sie Merkmalen in dem zu bearbeitenden Signal besser entsprechen. Dies weicht von einer vorbestimmten temporalen oder spektralen Skalierung eines vorbestimmten festen Stamm-Wavelets ab, wie es für die Wavelet-Transformation gebraucht wird. Innerhalb der hier vorgestellten Vorrichtung kann die Anpassung des Vergleichssignals alle Aspekte der Signalform betreffen und schreitet mit fortlaufender Verarbeitung des Meßsignals und der entsprechenden Berechnung der Korrelationskoeffizienten fort. Somit hat das Vergleichssignal in der Vorrichtung mit einem Wavelet im wesentlichen nur gemein, daß sein Integral über die Zeit Null ist oder entsprechend die Summe seiner diskreten Zeitwerte Null. Bei Bedarf können weitere Beschränkungen für die Form des Vergleichssignals vorgesehen werden.

Folgende Grundsätze gelten für die Korrelationsfunktionen, wie sie in der vorgestellten Vorrichtung verwendet werden:

1. Die Berechnung der Korrelationsfunktion wird im Zeitbereich ausgeführt.

2. Die Korrelationsfunktion eines Eingangs- oder Meßsignals besitzt die gleiche Zeitskala wie das Meßsignal.

3. Die Korrelationsfunktion hat einen bestimmten Wert für jeden diskreten Wert des Meßsignals.

4. Die Korrelationsfunktion beruht auf einem Vergleichssignal (KorrelatorWavelet), welches ebenfalls eine Funktion der Zeit ist.

5. Das Integral des Vergleichssignals über die Zeit sollte Null sein; im Falle eines zeitdiskreten Vergleichssignals sollte die Summe der Signalwerte Null sein.

6. Das Vergleichssignal kann von einem bekannten oder erwarteten Merkmal des Meßsignals abgeleitet werden. In diesem Fall wird die Korrelationsfunktion immer dort Maxima aufweisen, wo das Merkmal in dem Meßsignal enthalten ist, einschließlich solcher Orte im Meßsignal, an denen das Merkmal verrauscht ist.

7. Jeder Wert der Korrelationsfunktion gleicht dem Korrelationskoeffizienten zwischen dem Meßsignal zu dem entsprechenden Zeitpunkt und dem Vergleichssignal. Auf diese Weise zeigt der Wert der Korrelationsfunktionen für jeden Zeitpunkt, wie stark das Meßsignal zu diesem Zeitpunkt dem Vergleichssignal ähnelt.

Entsprechungen von Korrelationsfunktionen im Frequenzbereich:

1. Jede Signalverarbeitung im Zeitbereich hat ein Äquivalent im Frequenzbereich.

2. Die Anwendung der Korrelationsfunktion im Zeitbereich entspricht einer Bandpaßfilterung. Weil das Integral des Vergleichssignals Null ist, ist die Gleichstromverstärkung dieses äquivalenten Filters Null.

3. Der Bandpaßfilter, der der Korrelationsfunktion entspricht, ist ein digitaler FIR-Filter (FIR = finite impulse response), in dem die Zahl der Koeffizienten der Anzahl der diskreten Signalwerte entspricht, die das Vergleichssignal bilden.

4. Der der Korrelationsfunktion entsprechende Bandpaßfilter ist für die Anzahl der Signalwerte des Vergleichssignals ein Optimalfilter für solche Merkmale innerhalb des Meßsignals, welche dem Vergleichssignal ähneln.

5. Der der Korrelationsfunktion entsprechende Bandpaßfilter wird automatisch angepaßt, wenn Details des Vergleichssignals modifiziert werden, wie bei der Anpassung an Variationen von bekannten, gemessenen Merkmalen des Meßsignals.

Das Meßsignal wird in dem ersten Speicher 22 zumindest zwischengespeichert. Der Zwischenspeicher kann dabei ein FIFO-Speicher sein und erlaubt es, das gemessene Signal nicht nur in Echtzeit zu verarbeiten, sondern auch später. Die Größe des ersten Speichers oder Zwischenspeichers 22 hängt vom Verhältnis der Abtastrate zur Prozessorgeschwindigkeit ab und wird weiterhin von den statistischen Eigenschaften des Meßsignals beeinflußt. Typischerweise wird der erste Speicher oder Zwischenspeicher 22 für ein EKG-Signal so zu bemessen sein, daß er die Meßwerte eines Signals von ungefähr einer Sekunde Länge aufnehmen kann. Die Verwaltung des ersten Speichers 22 erfolgt über einen Index-Zeiger, wie er weiter unten beschrieben ist.

Von besonderer Bedeutung ist die Auswahl des zunächst vorzugebenden Vergleichssignals iWav. Wichtige Parameter sind diesbezüglich die Länge des Vergleichssignals und dessen Form.

Die Länge des Vergleichssignals, wLen, beeinflußt direkt sowohl die größte Breite (zeitliche Ausdehnung) der zu detektierenden Merkmale als auch die erforderliche Rechenkapazität. Bei der Simulation einer Vorrichtung zur Verarbeitung eines Meßsignals einer Herzkammer mit einer Abtastrate von 250 Hz entsprechend einem Zeitraster von 4 ms hat sich eine Vergleichssignallänge von 16 Signalwerten als geeignet erwiesen. In zeitlicher Hinsicht entspricht dies einer Dauer des Vergleichssignals von 64 ms. Wenn jedoch ein einziger EKG-Kanal Signale zweier Kammern enthält, wie beispielsweise aus dem rechten und dem linken Atrium, können zusätzliche Vergleichssignale erforderlich sein, um die kombinierten Merkmale abzudecken.

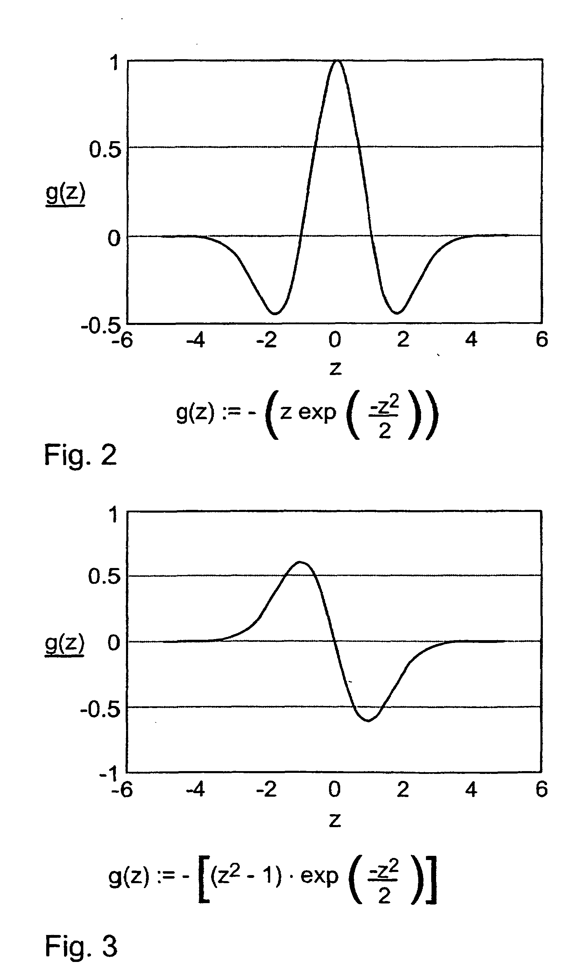

Von großer Bedeutung ist auch die zunächst vorgegebene Form des Vergleichssignals, welches in dem Speicher 26 abgelegt ist. Neben diesem einen Vergleichssignal können auch weitere Vergleichssignale in der Datenbank 40 abgelegt sein. Die Form des mindestens einen Vergleichssignals wird durch Schätzen der Signalform desjenigen Signalmerkmals in dem Meßsignal festgelegt, welches vorrangig detektiert werden soll. Alternativ kann das Vergleichssignal aber auch eine Standardform haben, wie die in den Figuren 2 oder 3 abgebildeten Wavelets vom Typ "Mexikanerhut" oder vom asymmetrischen Typ. Falls solch ein Standardwavelet als Vergleichssignal verwendet wird, sollte dessen Zeitskalierunng ungefähr der Dauer des erwarteten Merkmals entsprechen. In jedem Fall sollte das anfänglich gewählte Vergleichssignal der Dynamik des Meßsignals entsprechen. Außerdem sollte die Summe der Signalwerte des Vergleichssignals Null sein.

In interessanten Anwendungsfällen der Erfindung wird die Vorrichtung benutzt, vergleichbare Signalmerkmale direkt zu messen. Ein solches Beispiel ist weiter unten beschrieben und betrifft das Messen der Zeitzusammenhänge zwischen Zweikammer-Elektrokardiogrammen. Für diesen Zweck kann das anfängliche Vergleichssignal, iWav, eine besondere Form haben und die Berechnung der Korrelationskoeffizienten kann für Signale einer Vielzahl von Datenkanälen erfolgen. Der Betrieb der Vorrichtung mit einem anfänglich gesetzten Vergleichssignal wird im Rahmen des klinischen Einsatzes allgemein dann vorkommen, wenn solch eine Vorrichtung implantiert wird. Nachfolgende Behandlungen werden normalerweise unter Verwendung einer angepaßten Version des ursprünglich gesetzten Vergleichssignals erfolgen, welches die Eigenschaften vorliegender EKG- oder IDZ-Signale eines Patienten widerspiegelt. Die Signalverarbeitung kann jedoch automatisch zur Verwendung des ursprünglich gesetzten Vergleichssignals zurückkehren und zwar im Rahmen einer Strategie der Wiedergewinnung von Daten im Falle eines Detektionsverlustes, daß heißt wenn keine Signalmerkmale mehr detektiert werden. Dies kann vorkommen, wenn sich die Morphologie des Meßsignals innerhalb kurzer Zeit stark ändert.

Eine besondere Eigenschaft der Vorrichtung ist ihre Fähigkeit zur Anpassung des Vergleichssignals an lokalisierte oder detektierte Signalmerkmale des Meßsignals.

Der Grad der Anpassung des Vergleichssignals wird durch den Parameter $\alpha$ gesteuert, welcher in der Vorrichtung gespeichert ist. Als geeignete Werte von $\alpha$ haben sich solche in der Größenordnung von 1/16 erwiesen. Dies ist deshalb vorteilhaft, weil die Multiplikation mit 1-$\alpha$ (siehe unten) auf einfache Weise durch Bitverschieben und Subtraktion in Form einer Shift-and-Subtract-Operation implementiert werden kann.

Wenn eine Anpassung des Vergleichssignals erforderlich ist, wird das Vergleichssignal wWav durch eines ersetzt, welches die Summe ist aus dem zu setzenden Vergleichssignal multipliziert mit (1-$\alpha$) und dem entsprechenden Signalwert des zuletzt lokalisierten Merkmals des Meßsignals multipliziert mit $\alpha$. Die daraus resultierende Anpassung zeigt eine exponentielle Gewichtung vorangegangener Merkmale. Die Anpassung des Vergleichssignals, welches im Zeitbereich definiert ist, entspricht im Frequenzbereich dem Anpassen der Form eines korrespondierenden Bandpaßfilters für die optimale Extraktion zu detektierender Merkmale des Meßsignals.

Obwohl die Anpassung des Vergleichssignals für jedes lokalisierte Merkmal innerhalb eines Herzzyklusses erfolgen kann, ist dies im allgemeinen nicht erforderlich. Vielmehr wird bevorzugt, die Anpassung zu vorgegebenen Zeitpunkten vorzunehmen, für deren Festlegung das physiologische Zeitverhalten der interessierenden Merkmale und der daraus zu extrahierenden Parameter zu berücksichtigen sind. Alternativ kann die Anpassung in Reaktion auf Änderungen der Höhe der lokalen Maxima der Korrelationsfunktion ausgelöst werden, welche sich bei der Lokalisation eines zu detektierenden Merkmals ergeben. Die Möglichkeit der Anpassung in jedem Herzzyklus kann jedoch im unmittelbaren Anschluß an die Implantation der Vorrichtung wertvoll sein und in anderen Situationen, wo eine schnelle Anpassung an vorliegende Meßsignale eines bestimmten Patienten gewünscht ist, insbesondere im Falle eines Detektionsverlustes.

Auch die angepaßten Vergleichssignale müssen die Bedingungen erfüllen, daß die Summe der diskreten Signalwerte, aus denen sich das Vergleichssignal zusammensetzt, Null ist. Diese Anpassung erfolgt in einem zweiten Adaptionsschritt und betrifft vorzugsweise die kleineren Signalwerte, wodurch der Einfluß auf die berechneten Werte des Korrelationskoeffizienten minimiert wird.

Vorzugsweise wird die dynamische Bandbreite des angepaßten Vergleichssignals wWav an die durch den Meßkanal bestimmte maximale Amplitude des Meßsignals angepaßt. Mit anderen Worte wird die Dynamik des Vergleichssignals auf die maximale Dynamik des Meßsignals normiert. Dies vermeidet eine quadratische Verzerrung einer Skala für einen Schwellwert für den Korrelationskoeffizienten, welcher ansonsten mit Variationen der Meßsignalamplitude einherginge.

Auch der Schwellwert für den Korrelationskoeffizienten, der für die Lokalisation von zu detektierenden Signalmerkmalen verwendet wird, wird vorzugsweise durch die Vorrichtung im Laufe der Zeit an die tatsächlichen Gegebenheiten angepaßt.

Die Anpassung des Schwellwertes detThr wird durch einen den Anpassungsgrad beschreibenden Parameter $\beta$ sowie durch eine Grenzwertasymptote $\gamma$ bestimmt. Sowohl $\beta$ als auch $\gamma$ sollen Werte < 1 haben und sind in einem Speicher des Gerätes gespeichert. Simulationen haben ergeben, daß brauchbare Werte für $\beta$ in der Größenordnung von ⅛ liegen, während brauchbare Werte für $\gamma$ eine Größenordnung von ½ haben. In der Simulation erwiesen sich diese Werte deshalb als brauchbar, weil sie eine Merkmalsdetektion auch in Gegenwart substantieller Änderungen des EKG's von Herzzyklus zu Herzzyklus zuließen. Wie bei der Anpassung des Vergleichsmusters wWav erlauben diese Werte für $\beta$ und $\gamma$ eine vorteilhafte Implementierung in Form einer Shift-and-Substract Operation.

Wenn eine Anpassung des Schwellwertes erforderlich ist, wird der augenblickliche Wert von detThr durch einen ersetzt, der sich wie folgt zusammensetzt: ursprünglicher Schwellwert multipliziert mit (1-$\beta$) und demjenigen Maximalwert des Korrelationskoeffizienten, der dem zuletzt lokalisierten Merkmal zugeordnet ist, multipliziert mit $\beta$ und $\gamma$. Eine derartige Anpassung des Schwellwertes führt zu einer exponentiellen Gewichtung vorangegebener Werte und zu einer asymptoptischen Anpassung an einen vorbestimmten Bruchteil $\gamma$ des Maximums des Korrelationskoeffizienten, welcher dem zuletzt lokalisierten Merkmal des EKG's oder IDZ's zugeordnet ist.

Generell wird bevorzugt, diese Anpassung des Schwellwertes nach jeder Lokalisation eines Signalmerkmales vorzunehmen. Dieses vermindert die Wahrscheinlichkeit ein Merkmal deshalb nicht zu erkennen, weil sich die Amplitude des Merkmals ändert und verursacht nur wenig zusätzlichen Berechnungsaufwand. Wie im Falle der Anpassung des Vergleichsmusters wWav können jedoch auch für die Anpassung des Schwellwertes verschiedene Strategien angewandt werden, die den Zeitpunkt des Anpassens des Schwellwertes beispielsweise von der Abweichung des jeweiligen Maximums des Korrelationskoeffizienten von demjenigen abhängig machen, welcher einem zuvor detektierten Merkmal zugeordnet ist.

Von großer Bedeutung für die Vorrichtung sind weiterhin die Methoden zur Verringerung des Rechenaufwands. Dies gilt insbesondere vor dem Hintergrund, daß sämtliche interne Vorgänge eines implantierten Gerätes so wenig Energie kosten sollen wie möglich. Dementsprechend umfaßt die Vorrichtung Mittel, um beispielsweise den Rechenaufwand für die Berechnung von Korrelationsfunktionen minimieren. Häufig wird dieser Rechenaufwand von einem Mikroprozessor geleistet. Im folgenden werden die grundlegenden Überlegungen und die Einzelheiten der Implementierung beschrieben.

Eine wichtige Einflußgröße auf den Rechenaufwand ist die Abtast- oder Samplingrate.

Die Berechnung einer Korrelationsfunktion über ein vorgegebenes Zeitintervall schließt für jeden abgetasteten Signalwert des Meßsignals die Multiplikation und das Aufsummieren benachbarter Abtastwerte und der Signalwerte und des Vergleichssignals ein. Wie zuvor beschrieben, wird das Vergleichssignal so gewählt, daß es ein kurzes, interessierendes Zeitintervall abdeckt. Die Abtastrate bestimmt die Anzahl der Abtast- oder Meßsignalwerte während des interessierenden Zeitintervalls, genauso wie die Anzahl der Signalwerte des Vergleichssignals. Dies hat zur Folge, daß die Zahl der Multiplikations- und Addierschritte für das gewählte Zeitintervall des Meßsignals mit dem Quadrat der Abtastrate steigt. Aus diesem Grunde ist es erforderlich diejenige Mindestabtastrate zu bestimmen, die noch ein brauchbares Ergebnis liefert, und diesen Wert nicht signifikant zu überschreiten. Die Bestimmung dieser Abtastrate kann wirkungsvoll mit Hilfe von Computersimulationen durchgeführt werden, in denen eine Vielzahl von bereits vorliegenden Meßsignalen verwendet werden, die bereits an Patienten aufgenommen wurden und in Datenbanken gespeichert sind. Wenn dies für die Merkmalserkennung und Ortung (Lokation) sowie für die hierin beschriebenen Analysen durchgeführt wird, zeigen die Ergebnisse, daß Abtastraten bei oder unterhalb von 250 Hz angemessen sind. Bestimmte Anwendungen können höhere Abtastraten erfordern, beispielsweise wenn das Interesse hochfrequenten Signalmerkmalen gilt.

Der Energieverbrauch jeder Berechnung isttypischerweise eine lineare Funktion der Rechengenauigkeit. Aus diesem Grunde ist es erforderlich, die minimale Rechengenauigkeit zu bestimmen, die brauchbare Ergebnisse liefert, und diesen Wert nicht deutlich zu übersteigen. Auch diese Bestimmung der minimalen Rechengenauigkeit kann wie zuvor beschrieben mit Hilfe von Computersimulationen effizient durchgeführt werden. Diese haben ergeben, daß eine Auflösung von größer als 8 Bit nicht erforderlich ist. Dies entspricht einer Dynamik oder Bandbreite von 256 Werten.

In der hier vorgestellten Vorrichtung wird die Korrelationsfunktion verwendet, um interessierende Signalmerkmale in einem EKG- oder IDZ-Signal zu detektieren und genau zu lokalisieren oder um spezifische Signalformanalysen und Vergleiche durchzuführen. Die zuvor angesprochenen Computersimulationen haben ergeben, daß in der Praxis sowohl die numerische Auflösung als auch die Genauigkeit der Korrelationsberechnung substantiell begrenzt werden können, ohne die Aussagekraft der Ergebnisse zu beeinträchtigen. Dieses bildet die Grundlage für verschiedene Mittel zur Begrenzung der Rechenlast, die im folgenden beschrieben werden.

Die Auswertung einer Korrelationsfunktion erfordert das Bilden eines Produktes von aufeinanderfolgenden Signalwertpaaren, die von einem Signalwert des Meßsignals und einem Signalwert des Vergleichssignals gebildet werden. Aufführungsformen der Vorrichtung, die beispielsweise eine 8-Bit-Darstellung der Signalwerte und eine Zweierkomplementnummerierung verwenden, würden üblicherweise die Bildung eines 16-Bit-Produktes erfordern. Tatsächlich ist in einem solchen System jedoch ein 8-Bit-Produkt ausreichend für die Merkmalsdetektion und Lokation. Dies entspricht der Trunkierung derjenigen Bits des Produktes, die Größenordnungen im Bereich von 0 bis 128 repräsentieren. Vor diesem Hintergrund ist zulässig auch an alternative Multiplikationsverfahren mit begrenzter Auflösung zu denken, welche die Rechenbelastung reduzieren können, unabhängig davon, ob diese Multiplikationsroutinen in Hardware oder in Form von Microcode eines Prozessors implementiert sind.

Eine wirkungsvolle Methode dieser Art verwendet eine kurze Tabelle, die in einem Speicher, dem Logarithmenspeicher 28, der Vorrichtung abgelegt ist und Integerwerte enthält, die Logarithmen von Zahlen entsprechen. In einem 8-Bit-Zweierkomplementsystem hat die Tabelle beispielsweise 129 Zellen, die den Zahlen m von 0 und 128 zugeordnet sind. Jeder Eintrag in der Tabelle enthält denjenigen Integerwert, der $\ln(m)* 128/\ln(128)$ am nächsten kommt. Nur der Eintrag für m=0 ist auf Null gesetzt. Auf diese Weise skaliert, decken die Einträge in der Logarithmentabelle den Bereich von 0 bis 128 ab.

Diese Tabelle wird auf die folgende Weise benutzt, um ein skaliertes Produkt von x und y mit begrenzter Auflösung zu bilden. Wie üblich repräsentieren |..| Absolutwerte:

1. lese den Inhalt der mm |x| zugeornten Zelle der Tabelle

2. lese den Inhalt der m = |y| zugeordneten Zelle der Tabelle

3. addiere die beiden Tabelleneinträge

4. subtrahiere 128

5. suche diejenige Zelle der Tabelle, deren Eintrag dem berechneten Wert am nächsten kommt

6. nimm denjenigen Wert m, dem die so gefundene Zelle zugeordnet ist als Ergebnis der Multiplikation

7. versieh das Ergebnis mit einem Vorzeichen entsprechend der Vorzeichen von x und y

Der vierte Schritt führt zu einem Skalierungsfaktor von 1/128, der die Verwendung einer üblichen Tabelle sowohl für den Logarithmus als auch für die Umkehrfunktion des Logarithmus erlaubt. Auf diese Weise kann das Produkt von x und y mit begrenzter Auflösung durch zwei Speicherleseschritte, eine Addition, eine Subtraktion und eine sechsstufige binäre Suche gefunden werden.

Die für die hier beschriebene Vorrichtung verwendeten Signalverarbeitungsmethoden zur Bildung der Korrelationsfunktion setzen das Vorhandensein lokal herausragender Werte des Korrelationskoeffizienten voraus, die sich aus der Ähnlichkeit des zu verarbeitenden EKG- oder IDZ-Signals mit dem Vergleichssignal ergeben. Im Allgemeinen treten diese lokalen Maxima des der Korrelationsfunktion entsprechenden Signals dann auf, wenn große Signalwerte des EKG- oder IDZ-Signals mit großen Werten der Vergleichsfunktion zusammentreffen. Wie zuvor erläutert, hat sich die Brauchbarkeit eines derartigen Vorgehens als relativ unabhängig von der absoluten Genauigkeit der Berechnung der Korrelationskoeffizienten erwiesen. Daher besteht eine weitere wirkungsvolle Methode zur Reduzierung der Rechenlast darin, von der Berechnung der Korrelationskoeffizienten solche Produkte auszunehmen, die Signalwerte des EKG- oder IDZ-Signals als Faktor enthalten, welche innerhalb eines engen Bandes in der Nähe des Mittelwertes des Signals liegen. Die erwähnten Computersimulationen haben ergeben, daß durch solches Vorgehen bei einem 8-Bit-Datensystem mit einer Abtastrate von 250 Hz eine 50%ige Einsparung der Rechenlast erreicht werden kann, wenn bei einem 8-Bit-Signal von den 256 möglichen Signalwerten solche von der Produktberechnung ausgenommen werden, die in ein Band der Breite von 20 Signalwerten fallen. Unter diesen Bedingungen behalten die resultierenden angepaßten Vergleichssignale eine zuverlässige Übereinstimmung mit entsprechenden Vergleichssignalen, die ohne Auslassung der mittleren Meßsignalwerte berechnet wurden.

In ähnlicher Weise können zur Reduzierung der Rechenlast solche Produkte von der Berechnung der Korrelationskoeffizienten ausgenommen werden, die Signalwerte des Vergleichssignals als Faktor enthalten, welche in ein enges Band nahe dem Mittelwert des Vergleichssignals fallen. Um in diesem Falle die Bedingung einzuhalten, daß die Summe aller Signalwerte des Vergleichssignals Null ist, können Signalwerte nahe dem Mittelwert bereits bei der Anpassung oder Bildung des Vergleichssignals nach der Normierung auf Null gesetzt werden.

Für die Merkmalsdetektion geben die Schwellwertvergleichsmittel 32 nur dann ein Erkennungsignal aus, wenn das aus den Korrelationskoeffizienten bestehende Korrelations-Signal zunächst oberhalb des Schwellwertes liegt und anschließend einen Nulldurchgang zeigt. Zu diesem Zeitpunkt ist der genaue Ort dieser Ereignisse nicht von kritischer Bedeutung, weil ihr Auftreten eine genaue Merkmalslokation durch den Lokationsdetektor 38 auslöst. Daher und in Anbetracht der üblichen Struktur von EKG- und IDZ-Signalen ist es akzeptabel, wenn zur Bildung des Erkennungssignals nur jeder zweite Korrelationssignalwert verarbeitet wird, wodurch eine 50%ige Reduktion der Rechenlast erzielt wird.

Bei Anwendung der hier beschriebenen Vorrichtung wird es regelmäßig vorkommen, daß interessierende Signalmerkmale eine relativ lange Dauer haben und sich nur langsam verändern. Ein Beispiel hierfür sind die eine Repolarisation darstellenden T-Wellen in einem EKG-Signal. In einem System mit einer Abtastrate von 250 Hz und einer angenommenen Länge des Vergleichssignals von 16 Signalwerten entspricht die Dauer des Vergleichssignals 64 ms des EKG-Meßsignals. Dies ist nicht geeignet für die zuverlässige Lokation von Merkmalen, welche sich über 100 ms oder mehr erstrecken, wie die zuvor genannten T-Wellen. Eine mögliche Lösung diese Problems wäre die Verlängerung des Vergleichssignals. Dieses bedeutet, daß das Vergleichssignal mehr Signalwerte umfaßt, so daß die Rechenbelastung steigt. Eine wirkungsvolle Alternative besteht darin, nur jeden n-ten Signalwert des Meßsignals für die Berechnung heranzuziehen, falls bekannt ist, daß das gesuchte Signalmerkmal wenig brauchbare Informationen in einem Frequenzbereich aufweist, der der Abtastrate geteilt durch 2n entspricht. Auf diese Weise kann der Verarbeitungsaufwand für den Fall wirkungsvoll reduziert werden, in dem langandauernde, sich langsam ändernde Merkmale von Interesse sind.

Im folgenden soll die Arbeitsweise der Vorrichtung zur Merkmalsdetektion und Lokation in EKG- oder IDZ-Signalen beschrieben werden.

Die Arbeitsweise beruht darauf, Korrelationskoeffizienten zwischen einander überlappend aufeinanderfolgenden kurzen Abschnitten des Meßsignals und einem Vergleichssignal zu bilden, dessen Länge den Abschnitten des Meßsignals entspricht. Durch das abschnittsweise Vergleichen des Meßsignals mit dem Vergleichssignal entsteht eine Folge von Korrelationskoeffizienten, von denen jeder genau einem Abschnitt des Meßsignals zugeordnet ist und die zusammen ein aus diskreten Korrelationswerten bestehendes Korrelationssignal ergeben.

Das Kriterium für die Merkmalsdetektion erfordert, daß zumindest ein Korrelationskoeffizient einen vorgegebenen positiven Schwellenwert überschreitet und das hierauf später ein Nulldurchgang des Korrelationssignals folgt. Wenn diese beiden Bedingungen erfüllt sind, gilt ein Merkmal als grob erkannt. Anschließend wird unter all denjenigen Korrelationskoeffizienten, die die vorgenannten Bedingungen erfüllen, der größte gesucht. Dieses lokale Maximum des Korrelationssignals bezeichnet dann die Lokation des zu detektierenden Signalmerkmals. Ist die Lokation einmal gefunden, wird sie zur weiteren Analyse in einem Speicher der Vorrichtung gespeichert. Über die zuvor genannte Zuordnung eines jeden Korrelationskoeffizienten des Korrelationssignals zu genau einem Abschnitt des Meßsignals ist durch die Lokation genau ein Abschnitt des Meßsignals bezeichnet, der in dem Bereich, in dem die Detektionsbedingungen erfüllt sind, die größte Ähnlichkeit mit dem Vergleichssignal aufweist. Somit kann der Ort eines Signalmerkmales in dem Meßsignal auf einen Abtastschritt genau bestimmt werden. Bei einer Abtastrate von 250 Hz heißt dies, daß die Lokation eines Signalmerkmals auf 4 ms genau erfolgt.

Die Form des Vergleichssignals sowie auch der Detektionsschwellenwert werden laufend angepaßt, um Änderungen der Meßsignalform Rechnung zu tragen. Die Art und Weise der Anpassung wurde zuvor beschrieben. Die Anpassung des Vergleichssignals an ein Meßsignal ermöglicht es, ein Signalmerkmal mit großer Wahrscheinlichkeit und möglichst genau zu erkennen und zu lokalisieren, und zwar auch dann, wenn das Meßsignal Schwankungen unterworfen ist oder beispielsweise mit Rauschen überlagert ist.

Für die folgende Beschreibung des Arbeitsablaufes in der Vorrichtung ist insbesondere der Zugriff auf den ersten Speicher 22, der die einzelnen Meßwerte enthält, die das Meßsignal oder zumindest Abschnitte davon repräsentieren, von Bedeutung. Der Zugriff auf den ersten Speicher 22 erfolgt mit Hilfe eines Indexzeigers, bIndex. Dieser Indexzeiger zeigt zunächst auf eine Speicherstelle, die soviele Speicherstellen von der ersten Speicherstelle 22 entfernt ist, wie es der Anzahl der Signalwerte entspricht, die das Vergleichssignal bilden. Die Anzahl dieser Signalwerte wird mit wLen bezeichnet. Wenn ein neuer Meßwert für das Meßsignal in den ersten Speicher geschrieben wird, bevor die Verarbeitung eines vorangegangenen Signalwertes vollendet ist, zeigt der Zeiger bIndex auf den vorangegangenen Meßsignalwert.

Die Signalverarbeitung in der Vorrichtung läuft wie folgt ab:

1. Zunächst wird in den Speicher für das Vergleichsmuster wWav ein vorgegebenes Vergleichsmuster iWav eingeschrieben. Dies ist die Initialisierung des Vergleichsmusters.

2. Die Initialisierung des Schwellwertes detThr für die Merkmalsdetektion geschieht, in dem dieser Schwellwert auf einen anfänglichen Schwellwert iDetThr gesetzt wird und dieser Wert in den Schwellwertspeicher 36 geschrieben wird. Vorzugsweise ist der Anfangsschwellwert iDetThr ein relativ kleiner Schwellwert, der sicherstellt, daß überhaupt ein Signalmerkmal erkannt wird. Ein typischer Wert für ein System, das mit einem 8-Bit-Meßsignal arbeitet und bei dem die Anzahl der Signalwerte des Vergleichssignals, wLen 16 ist, ist 20 für den Schwellwert.

3. Anschließend wird das Erkennungsignal, detState auf "0" gesetzt. Das Erkennungssignal kann insgesamt drei Zustände annehmen, von denen der Zustand "0" der erste ist und den Zustand kennzeichnet, in dem die Merkmalsdetektion nicht aktiv ist.

4. Der Wert des größten Korrelationskoeffizienten in einem Abschnitt des Korrelationssignals, das die zuvor beschriebenen Detektionsbedingungen erfüllt, heißt maxccVal. Dieser Wert wird bei der Initialisierung auf den größten zugelassenen negativen Wert gesetzt.

5. Der Zeitpunkt zu dem der Wert maxccVal in Bezug auf das Meßsignal auftritt, als "0" die zeitliche Lokation des lokalen Maximums des Korrelationssignals, wird mit maxccLoc bezeichnet und bei der Initialisierung auf "0" gesetzt.

6. Für jeden Meßsignalwert des Meßsignals, der an der durch bIndex bezeichneten Speicherstelle des ersten Speichers 22 steht, wird das Vergleichssignal wWav verwendet, um den Korrelationskoeffizienten ccVal über die Anzahl wLen der das Vergleichssignal bildenden Meßsignalwerte gebildet, die dem durch bIndex bezeichneten Meßsignalwert vorangehen, daß heißt die vor diesem Meßsignalwert aufgenommen wurden.

7. Wenn ccVal größer ist als maxccVal, wird der Wert von maxccVal auf den Wert von ccVal gesetzt und der Ort des entsprechenden Signalwertes des Meßsignales als maxccLoc gespeichert.

8. Wenn der Wert von ccVal größer ist als der von detThr, und wenn gleichzeitig detState "0" ist, wird detState auf "1" gesetzt. Das Erkennungsignal hat somit den Wert "1". Dies bezeichnet, daß die Signalerkennung nun aktiv ist.

9. Wenn der Wert ccVal kleiner ist als Null, und der Zustand des Erkennungssignals, detState "1" ist, wird dieser Zustand detState auf "2" gesetzt. Der Zustand "2" des Erkennungssignals bezeichnet, daß das Meßsignal nun die Detektionsbedingungen erfüllt, daß heißt, daß ein interessierendes Signalmerkmal detektiert ist. Im nächsten Schritt wird die Lokation dieses Merkmals so genau wie möglich bestimmt. Dies geschieht, in dem der größte Wert von ccVal für die folgenden, der Anzahl wLen der Signalwerte des Vergleichssignals entsprechenden Meßsignalwerte gesucht wird, die auf den mit maxccLoc bezeichneten Meßsignalwert folgen:

10. Wenn der Zustand des Erkennungsignals, detState 2 ist, werden die folgenden Schritte durchgeführt, ansonsten wird zum Schritt 6 zurückgekehrt:

10.1 Verwende das Vergleichssignal wWav, um den Korrelationskoeffizienten ccVal über die der Anzahl wLen der Signalwerte des Vergleichssignals entsprechende Zahl der Meßsignalwerte zu berechnen, die auf den mit maxccLoc bezeichneten Meßsignalwert folgen.

10.2 Falls sich bei einem der Schritte gemäß 10.1 ergibt, daß ccVal den Wert von maxccVal überschreitet, wird der Wert von maxccVal auf den Wert von ccVal gesetzt und der Ort des entsprechenden Meßsignalwertes als featureLoc gespeichert. Der endgültige Wert von featureLoc repräsentiert dann den genauesten Wert für das zeitliche Auftreten eines zu detektierenden Merkmals, bildet also einen Zeitstempel für dieses Merkmal, sobald sich das Vergleichssignal wWav an die Meßsignalstruktur angepaßt hat.

10.3 Anschließend wird das lokalisierte Signalmerkmal und sein Zeitstempel für die weitere Analyse gespeichert.

10.4 Falls eine Vergleichssignalanpassung vorgesehen ist, wird nach der Lokation eines Signalmerkmals die Anpassung des Vergleichssignals wie folgt durchgeführt:

10.4.1
Bilde ein neues Vergleichssignal wWav mit dem Für einen vorgegebenen Vergleichsignalanpassungsparameter $\alpha<1$ im Speicher der Vergleichssignalanpassungseinheit 40 unter Berücksichtigung von wWav und dem lokalisierten Meßsignalabschnitt mit der Länge (Anzahl der Meßsignalwerte) wLen wie folgt: multipliziere die Signalwerte von wWav mit $(1-\alpha)$ und addiere hierzu die wLen entsprechende Zahl der Meßsignalwerte am Ort featureLoc, multipliziert mit $\alpha$.

10.4.2
Skaliere das Ergebnis so, daß das neue Vergleichssignal wWav die maximale Dynamik besitzt, die durch die Meßeinrichtung für das Meßsignal vorgegeben ist, so, daß die Summe der Signalwerte des Vergleichssignals Null ist. Schreibe das so gebildete neue Vergleichssignal wWav in den zweiten Speicher 26.

10.4.3
Bilde einen neuen Schwellwert auf Basis der Schwellwertanpassungsparameter $\beta$ und der Schwellwertasymptote $\gamma$ im Speicher der Schwellwertanpassungseinheit 34 wie folgt: multipliziere den ursprünglichen Schwellwert detThr mit $(1-\beta)$ und addiere hierzu $\beta * \gamma * maxccVal$. Speichere den so gewonnenen neuen Schwellwert im Schwellwertspeicher 36.

10.4.4
Speichere bei Bedarf das neugebildete Vergleichssignal wWav in einem Speicher für die weitere Analyse. Das angepaßte Vergleichssignal enthält die exponentielle gewichtete statistische Information über die zuletzt lokalisierten Signalmerkmale, die für einige Anwendungen von Interesse sein können.

10.5 Setze den Speicherindex bIndex an eine Position, die um tRef /tSamp Speicherstellen vor featureLoc liegt, wobei tRef die gewünschte Refraktärzeit des Meßkanals ist, während tSamp die Abtastrate ist. Hierbei können je nach Wahl, wie eingangs angedeutet, bestimmte Meßsignalwerte für die Berechnung des Korrelationskoeffizienten ausgelassen werden oder es kann die Verarbeitung verzögert werden, um eine Refraktärzeit zu implementieren. Der neugebildete Speicherindex bIndex sollte jedoch mindestens um eine wLen entsprechende Zahl von Speicherstellen vor der ersten Speicherstelle des Speichers 22 liegen, um die nächste Merkmalslokation einwandfrei durchführen zu können.

10.6 Kehre zu Schritt 3 zurück. Im Falle eines Detektionsverlustes kann eine vorbestimmte Strategie zur Wiedergewinnung der Daten angewandt werden, bevor zu Schritt 3 zurückgekehrt wird.

Im folgenden soll nun eine Strategie zur Datenwiedergewinnung beim Detektionsverlust beschrieben werden.
Ein Verlust der Merkmalserkennung kann aus verschiedenen Gründen auch außerhalb einer Klinik beispielsweise bei medizinischen Notfällen vorkommen. Wenn dieser Detektionsverlust für eine bestimmte Zeit anhält, daß heißt wenn eine zeitlang kein Signalmerkmal mehr erkannt wird, so daß auch eine Anpassung des Vergleichssignals und des Detektionsschwellwertes nicht mehr erfolgt, muß eine autonome Strategie vorgesehen werden, um den gewünschten Betrieb wieder herzustellen. Hierzu gibt es verschiedene Möglichkeiten, die gewisse Gemeinsamkeiten aufweisen:

1. setze detThr = iDetThr oder auf einen anderen vorgegebenen kleinen Wert

2. löse eine Vergleichssignalanpassung von Herzzyklus zu Herzzyklus aus oder

3. initialisiere das Vergleichssignal wWav neu

Ziel von 1, ist es, die Wahrscheinlichkeit einer Merkmalsdetektion in dem betroffenen Kanal zu maximieren. Ziel von 2. ist es, eine schnelle Anpassung des Vergleichssignals an detektierte Signalmerkmale sicherzustellen. Ziel von 3. ist es, ein Vergleichssignal vorzugeben, das für die Detektion einer großen Zahl möglicher Merkmale geeignet ist. Dieses Vergleichssignal kann dem ursprünglichen Vergleichssignal iWav entsprechen, wie es zuvor beschrieben wurde, oder es kann ein spezielles Vergleichssignal für den Fall eines Detektionsverlustes sein.

Im folgenden wird nun beschrieben, wie die Vorrichtung die bereits angesprochene Signalanalyse durchführt.

Ein Aspekt der Signalanalyse ist die Analyse der Zeitbeziehungen zwischen linkem und rechtem Atrium oder linkem und rechtem Ventrikel eines Herzens. Dem kann die Vorrichtung auf verschiedene Art und Weise dienen, je nachdem, welcher Art die Datenaufnahme in den verschiedenen Kammern ist. Die folgenden zwei Beispiele sollen dies erläutern.

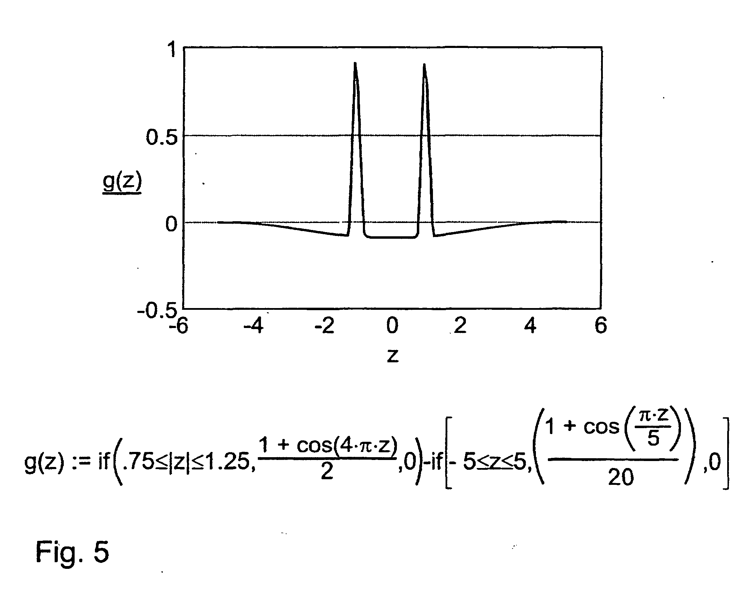

Wenn zwei separate Kanäle für die beiden Herzkammern zur Verfügung stehen, kann die Überleitungszeit beispielsweise vom rechten zum linken Atrium durch Verarbeitung einer Signalwertfolge geschehen, die sich aus Werten beider Signalaufnehmer zusammensetzt und mit den Signalwerten des rechten Atriums beginnt und mit denen des linken Atriums fortsetzt. Das vorgegebene Vergleichssignal ist bimodal und hat zwei Maximalwerte wie das Beispiel in Figur 5 zeigt. Der zeitliche Abstand dieser beiden Maximalwerte entspricht dem erwarteten Wert für die Überleitungszeit. Für die Berechung der Korrelationskoeffizienten werden zunächst die erste Hälfte der Signalwerte des Vergleichssignals verwendet, die das erste Maximum enthalten und mit denjenigen Meßsignalwerten verrechnet, die vom rechten Atrium stammen. Die zweite Hälfte der Signalwerte des Vergleichsmusters, die dessen zweites Maximum enthalten, werden dann mit Signalwerten verrechnet, die vom linken Atrium stammen. Das Ergebnis ist die Detektion eines künstlichen Merkmals, welches ein bimodales Kompositum aus den zwei Kanälen ist und Ereignisse enthält, die beiden Kammern zugeordnet sind. Die zuvor beschriebene Anpassung des Vergleichsmusters führt dazu, daß dieses zunehmend die duale Struktur des Kompositums widerspiegelt. Jedes Kompositum kann dann hinsichtlich des seitlichen Abstandes von rechts- und linksatrialen Ereignissen analysiert werden. Dies gilt für das Kompositum des Meßsignals. Wenn ein statistischer Mittelwert beispielsweise für die Überleitungszeit gesucht wird, kann anstelle eines Kompositums aus dem rechts- und linksatrialen Meßsignal auch das angepaßte Vergleichssignal analysiert werden. In beiden Fällen umfaßt die Analyse die Identifikation zweier lokaler Maxima und die Bestimmung von deren zeitlichem Abstand. Dieser zeitliche Abstand der lokalen Maxima entspricht der Überleitungszeit.

Falls für zwei Kammern nur ein Kanal zur Verfügung steht, kann die zuvor beschriebene Strategie nicht angewandt werden. Stattdessen muß jedes detektierte Merkmal des gemeinsamen Kanals analysiert werden, um den Zeitabstand individueller rechts- und linksatrialer Ereignisse zu bestimmen. Wie zuvor beschrieben, kann diese alternativ durch Analyse des Meßsignals oder durch Analyse des Vergleichssignals geschehen. Auch bei diesen Analysen kommt es darauf an, zwei lokale Maxima zu detektieren sowie deren zeitlichen Abstand. Die Analysetechniken können jedoch wegen der Überlagerungseffekte der Signale in einem gemeinsamen Meßkanal komplexer sein.

Neben der Bestimmung der Überleitungszeit besteht eine weitere Analyse in der Klassifikation detektierter Merkmale. Dies kann mit Hilfe verschiedener Vergleichssignale geschehen, die jeweils für eine bestimmte Signalklasse charakteristisch sind. Auch hierzu kann die Vergleichssignaldatenbank 42 dienen.

| Anhang | |
|---|---|
| α | Anpassungsgrad für die Vergleichssignalanpassung |
| β | Anpassungsgrad für die Detektionsschwellwertanpassung |
| γ | Anpassungsasymptote für den Detektionsschwellwert |
| bindex | Indexzeiger für den ersten Speicher 22 |
| ccVal | Wert eines Korrelationskoeffizienten |
| maxccVal | maximaler lokaler Korrelationskoeffizient in dem Abschnitt des Korrelationssignals, für den die Bedingungen der Merkmalsdetektion erfüllt sind |

(fortgesetzt)

| Anhang | |
|---|---|
| maxccLoc | zeitliche Lokation von maxccVal |
| feature-Loc | zeitliche Lokation eines lokalisierten Merkmals |
| detState | logischer Zustand des Erkennungssignals |
| detThr | Wert des Detektionsschwellwertes |
| iDetThr | ursprünglicher, vorgegebener Wert des Detektionsschwellwerts |
| tRef | gewünschte Refraktärzeit des Meßkanals |
| tSamp | Abtastrate für das Meßsignal |
| wLen | Anzahl der Signalwerte des Vergleichssignals |
| wWav | angepaßtes Vergleichssignal |
| iWav | ursprüngliches, vorgegebenes Vergleichssignal vor der Anpassung |

**Patentansprüche**

1. Vorrichtung zur Verarbeitung von Körpersignalen, mit wenigstens einem Sensor (10, 12) zur Aufnahme insbesondere elektrischer Signale eines lebenden Körpers sowie mit Mitteln (16, 18) zur Aufbereitung aufgenommener Signale für die weitere Verarbeitung und wenigstens einem ersten Speicher (22) für ein aufgenommenes Messsignal oder Signalabschnitte davon, mit mindestens einem zweiten Speicher (26), welcher ein vorgebbares, zeitlich endliches Vergleichssignal enthält, sowie durch Signalvergleichsmittel (24), die mit dem zweiten (26) und dem ersten Speicher (22) verbunden und zum gleitenden Vergleichen sich zeitlich überlappender Signalabschnitte des Messsignals im ersten Speicher (22) mit dem in dem zweiten Speicher (26) gespeicherten Vergleichsignal und zur Ausgabe eines die Ähnlichkeit eines jeden verglichenen Signalabschnittes des Messsignals mit dem Vergleichssignal repräsentierenden Korrelationskoeffizienten ausgebildet sind, **dadurch gekennzeichnet, dass** das Integral des Vergleichssignals über die Zeit oder dessen Summe zeitlich diskreter Signalwerte Null ist.

2. Vorrichtung nach Anspruch 1, **dadurch gekennzeichnet, dass** die Signalvergleichsmittel (24) mit einem Logarithmenspeicher (28) verbunden sind, der Tabellen von Logarithmen für Werte des Mess- und des Vergleichssignals enthält, wobei die Signalvergleichseinheit zum Bilden der Korrelationskoeffizienten so ausgebildet sind, dass sie eine Multiplikation eines Wertes des Vergleichssignals aus dem zweiten Speicher (26) mit einem entsprechenden Wert des Messsignals aus dem ersten Speicher (22) derart durchführen, dass zunächst aus dem Logarithmenspeicher die Logarithmen der zu multiplizierenden Werte selbst oder der ihnen jeweils nächstkommenden Werte ausgelesen werden und anschließend die beiden Logarithmen addiert werden.

3. Vorrichtung nach Anspruch 1 oder 2, **gekennzeichnet durch** Detektionsmittel (30), die mit den Signalvergleichsmitteln (24) verbunden und zum Detektieren von Maximalwerten und/oder Nulldurchgängen eines von den Korrelationskoeffizienten gebildeten Signals ausgebildet sind.

4. Vorrichtung nach einem der Ansprüche 1 bis 3, **gekennzeichnet durch** Schwellwertvergleichsmittel (32), die mit den Signalvergleichsmitteln (24) und einem, einen Schwellwert enthaltenden Schwellwertspeicher (36) verbunden und zur Ausgabe eines Erkennungssignals ausgebildet sind, sobald der von den Signalvergleichsmitteln (24) ausgegebene Korrelationskoeffizient den Schwellwert überschreitet.

5. Vorrichtung nach Anspruch 4, **dadurch gekennzeichnet, dass** die Schwellwertvergleichsmittel (32) zusätzlich so ausgebildet sind, dass sie ein Erkennungssignal ausgeben, wenn ein Korrelationskoeffizient für einen ersten Signalabschnitt aus dem ersten Speicher (22) den Schwellwert überschreitet und für einen zweiten, zeitlich nach dem ersten Signalabschnitt aufgenommenen Signalabschnitt den Wert Null erreicht oder unterschreitet.

6. Vorrichtung nach Anspruch 3 und einem der Ansprüche 4 bis 5, **gekennzeichnet durch** Lokationsmittel (38) die mit den Schwellwertvergleichsmitteln (32) und den Detektionsmitteln (30) verbunden und so ausgebildet sind, dass sie demjenigen Signalabschnitt des Messsignals in dem ersten Speicher (22) ein Lokationssignal zuordnen, für den das von Korrelationskoeffizienten gebildete Signal ein Maximum innerhalb desjenigen Anschnittes des von den Korrelationskoeffizienten gebildeten Signals besitzt, für das die Schwellwertvergleichsmittel (32) ein Erkennungssignal ausgeben.

7. Vorrichtung nach Anspruch 6, **gekennzeichnet durch** Schwellwertbildungsmittel (34), die mit dem Schwellwertspeicher (36) und den Lokationsmitteln (38) verbunden und so ausgebildet sind, dass sie einen neuen Schwellwert nach Auftreten eines Lokationssignals derart bilden, dass der dem Lokationssignal zugeordnete Korrelationskoeffizient gewichtet in die Bildung des neuen Schwellwertes eingeht.

8. Vorrichtung nach einem der Ansprüche 1 bis 7, **gekennzeichnet durch** Vergleichssignalbildungsmittel zum Bilden eines neuen Vergleichssignals, welche mit dem zweiten Speicher (26) verbunden und so ausgebildet sind, dass ein gemessener Signalabschnitt, der einem zu detektierenden Signalmerkmal entspricht, derart zu dem Vergleichssignal transformiert wird, das sein Intergral über die Zeit Null ist, und dass so gebildete Vergleichsignal in den zweiten Speicher (26) übertragen wird.

9. Vorrichtung nach einem der Ansprüche 1 bis 8, **gekennzeichnet durch** Vergleichssignalanpassungsmittel (40) zum Anpassen des Vergleichssignals, welche mit dem ersten Speicher (22), dem zweiten Speicher (26) und den Lokationsmitteln (38) verbunden und so ausgebildet sind, dass sie eine neues, angepasstes Vergleichssignal bilden, wenn die Lokationsmittel (38) ein Lokationssignal ausgeben, wobei das angepasste Vergleichssignal unter Verwendung desjenigen Messsignalabschnittes aus dem ersten Speicher (22) gebildet wird, dem das Lokationssignal zugeordnet ist.

10. Vorrichtung nach Anspruch 9, **gekennzeichnet durch** derart ausgebildete Vergleichssignalanpassungsmittel (40), dass das vor dem Anpassen gültige Vergleichssignal zur Bildung des nach dem Anpassen gültigen Vergleichsignals mit einem Faktor 1-$\alpha$ gewichtet wird, während derjenige Signalabschnitt im ersten Speicher (22), der das Erkennungssignal mittels der Signalvergleichs- und der Lokationsmittel ausgelöst hat, mit einem Gewichtungsfaktor $\alpha$ in das nach dem Anpassen gültige Vergleichssignal eingeht.

11. Vorrichtung nach einem der Ansprüche 8 bis 10, **dadurch** gegenzeichnet, dass die Vergleichssignalbildungsmittel (40) und/oder die Vergleichssignalanpassungsmittel derart ausgebildet sind, dass das so gebildete bzw. angepasste Vergleichssignal derart normiert ist, dass dessen Amplitude der maximalen Amplitude des Messsignals entspricht.

12. Vorrichtung nach einem der Ansprüche 1 bis 11, **gekennzeichnet durch** eine Datenbank (42), welche mehrere Vergleichssignale enthält und derart mit dem zweiten Speicher (26) verbunden ist, dass Vergleichssignale von der Datenbank in den zweiten Speicher (26) und umgekehrt übertragbar sind.

13. Vorrichtung nach einem der Ansprüche 1 bis 12, **dadurch gekennzeichnet, dass** sie Analysemittel zur Analyse der kennzeichnenden Eigenschaften des vorzugsweise angepassten Vergleichssignals umfasst.

14. Vorrichtung nach einem der Ansprüche 1 bis 13, **gekennzeichnet durch** Mittel (10, 12) zum Aufnehmen zweier Herzsignale, von denen eines dem linken und das andere dem rechten Ventrikel bzw. Atrium zugeordnet ist, mit diesen Mitteln verbundene Mittel zum Bilden eines bimodalen Signals aus den zwei Herzsignalen, dergestalt, dass das bimodale Signal ein Merkmal des ersten Signals vor seiner Überleitung in das jeweils andere Ventrikel oder Atrium und das entsprechende Merkmal nach seiner Überleitung enthält, so dass das Merkmal in einem der Überleitungszeit entsprechenden zeitlichen Abstand einmal in seiner Form vor und einmal in seiner Form nach der Überleitung in dem bimodalen Signal enthalten ist, sowie **dadurch** dass der zweite Speicher ein bimodales Vergleichssignal enthält, welches an das bimodale Signal anpassbar ist, so dass nach Anpassung des bimodalen Vergleichsignal an das bimodale Signal die Überleitungszeit **durch** eine Analyse des Vergleichssignals bestimmbar ist.

## Claims

1. Apparatus for processing body signals, comprising at least one sensor (10, 12) for picking up, in particular, electrical signals from a living body, means (16, 18) for preparing for further processing picked-up signals, at least one first memory (22) for a picked-up measurement signal or portions thereof, at least one second memory (26), which contains a predeterminable comparison signal that is finite in respect of time, and signal comparing means (24), which are connected to the second memory (26) and the first memory (22) and are configured for sliding comparison of signal portions, which overlap in respect of time, of the measurement signal in the first memory (22) to the comparison signal stored in the second memory (26) and for output of a correlation coefficient representing the similarity of each compared signal portion of the measurement signal to the comparison signal, **characterised in that** the integral of the comparison signal in relation to time or its sum of the time-discrete signal values is zero.

2. Apparatus according to claim 1, **characterised in that** the signal comparing means (24) are connected to a logarithm memory (28) that contains tables of logarithms for values of the measurement signal and the comparison signal, the signal comparing means being configured for forming the correlation coeffcients such that they execute multiplication of a value of the comparison signal from the second memory (26) by a corresponding value of the measurement signal from the first memory (22) in such a way that firstly the logarithms of the values to be multiplied themselves or the values respectively closest thereto are read out of the logarithm memory and the two logarithms are then added.

3. Apparatus according to either claim 1 or claim 2, **characterised by** detection means (30), which are connected to the signal comparison means (24) and are configured for detecting maximum values and/or zero-passages of a signal formed by the correlation coefficients.

4. Apparatus according to any one of claims 1 to 3, **characterised by** means (32) for comparing threshold values, which are connected to the signal comparing means (24) and a threshold value memory (36) containing a threshold value and are configured for outputting an identification signal as soon as the correlation coefficient output by the signal comparing means (24) exceeds the threshold value.

5. Apparatus according to claim 4, **characterised in that** the threshold value comparing means (32) are additionally configured such that they output an identification signal when a correlation coefficient for a first signal portion from the first memory (22) exceeds the threshold value and a correlation coefficient for a second signal portion, which is recorded in respect of time after the first signal portion, reaches or is below the value zero.

6. Apparatus according to claim 3 and either claim 4 or claim 5, **characterised by** locating means (38), which are connected to the threshold value comparing means (32) and the detection means (30) and are configured such that they allocate a location signal to the signal portion of the measurement signal in the first memory (22) in respect of which the signal formed by correlation coefficients has a maximum within the portion of the signal formed by the correlation coefficients for which the threshold value comparing means (32) output an identification signal.

7. Apparatus according to claim 6, **characterised by** threshold value-forming means (34), which are connected to the threshold value memory (36) and the locating means (38) and are configured such that they form a new threshold value after the occurrence of a location signal in such a way that the correlation coefficient associated with the location signal is involved in weighted fashion in the formation of the new threshold value.

8. Apparatus according to any one of claims 1 to 7, **characterised by** means for forming a new comparison signal, which are connected to the second memory (26) and are configured such that a measured signal portion, which corresponds to a signal feature to be detected, is transformed to the comparison signal in such a way that its integral in relation to time is zero and the comparison signal thus formed is transferred into the second memory (26).

9. Apparatus according to any one of claims 1 to 8, **characterised by** comparison signal-adaptation means (40) for adaptation of the comparison signal, which are connected to the first memory (22), the second memory (26) and the locating means (38) and are configured such that they form a new adapted comparison signal when the locating means (38) output a location signal, the adapted comparison signal being formed using the measurement signal portion from the first memory (22) with which the location signal is associated.

10. Apparatus according to claim 9, **characterised by** comparison signal-adaptation means (40), which are configured such that, for forming the comparison signal valid after adaptation, the comparison signal valid prior to adaptation is weighted with a factor $1-\alpha$, while the signal portion in the first memory (22) which has triggered the identification signal by means of the signal comparison means and the locating means is involved with a weighting factor $\alpha$ in the comparison signal which is valid after adaptation.

11. Apparatus according to any one of claims 8 to 10, **characterised in that** the comparison signal-forming means (40) and/or the comparison signal-adaptation means are configured such that the comparison signal thus formed or adapted is standardised in such a way that the amplitude thereof corresponds to the maximum amplitude of the measurement signal.

12. Apparatus according to any one of claims 1 to 11, **characterised by** a database (42), which contains a plurality of comparison signals and is connected to the second memory (26) in such a way that comparison signals can be transferred from the database into the second memory (26) and *vice versa.*

13. Apparatus according to any one of claims 1 to 12, **characterised in that** it comprises means for analysis of the **characterising** properties of the preferably adapted comparison signal.

14. Apparatus according to any one of claims 1 to 13, **characterised by** means (10, 12) for picking up two cardiac signals, one of which is associated with the left ventricle or atrium and the other of which is associated with the right ventricle or atrium, means, connected to these means, for forming a bimodal signal from the two cardiac signals in such a way that the bimodal signal contains a feature of the first signal prior to conduction into the respective other ventricle or atrium and the corresponding feature after conduction thereof, such that the feature is contained in the bimodal signal at an interval in respect of time corresponding to the conduction time, on the one hand in its form prior to conduction and on the other hand in its form after conduction, and in that the second memory contains a bimodal comparison signal which can be adapted to the bimodal signal such that, after adaptation of the bimodal comparison signal to the bimodal comparison signal, the conduction time can be determined by analysis of the comparison signal.

## Revendications

1. Dispositif pour traiter des signaux émis par un corps vivant, comportant au moins un capteur (10, 12) pour enregistrer notamment des signaux électriques d'un corps vivant ainsi que des moyens (16, 18) pour traiter des signaux enregistrés pour le traitement ultérieur et au moins une première mémoire pour un signal de mesure enregistré ou des parties d'un tel signal, comportant au moins une seconde mémoire (26), qui contient un signal de comparaison fini dans le temps, pouvant être prédéterminé, ainsi que des moyens (24) de comparaison de signaux, qui sont reliés à la seconde mémoire (26) et à la première mémoire (22) et agencés pour réaliser la comparaison glissante de signaux du signal de mesure, qui se chevauchent dans le temps, dans la première mémoire (22) au signal de comparaison mémorisé dans la seconde mémoire (26) et pour délivrer un coefficient de corrélation qui représente la similitude de chaque partie comparée du signal de mesure avec le signal de comparaison, **caractérisé en ce que** l'intégrale du signal de comparaison dans le temps ou sa somme de valeurs du signal discrètes dans le temps est nulle.

2. Dispositif selon la revendication 1, **caractérisé en ce que** les moyens (24) de comparaison de signaux (24) sont reliés à une mémoire de logarithmes (28), qui contient des tables de logarithmes pour des valeurs du signal de mesure et du signal de comparaison, l'unité de comparaison de signaux pour la formation des coefficients de corrélation étant agencée de telle sorte qu'elle exécute une multiplication du signal de comparaison provenant de la seconde mémoire (26) par une valeur correspondante du signal de mesure provenant de la première mémoire (22), de telle sorte que tout d'abord l'algorithme des valeurs elles-mêmes devant être multipliées ou les valeurs, qui leur sont respectivement les plus proches, sont lues à partir de la mémoire de logarithmes et qu'ensuite les deux logarithmes sont additionnés.

3. Dispositif selon la revendication 1 ou 2, **caractérisé par** des moyens de détection (30), qui sont reliés aux moyens (24) de comparaison de signaux et sont agencés pour détecter des valeurs maximales et/ou des passages par zéro d'un signal formé par le coefficient de corrélation.

4. Dispositif selon l'une des revendications 1 à 3, **caractérisé par** des moyens (32) de comparaison à valeurs de seuil, qui sont reliés aux moyens (24) de comparaison de signaux et à une mémoire de seuil (36) contenant une valeur de seuil et sont conçus pour délivrer un signal d'identification, dès que le coefficient de corrélation délivré par les moyens (24) de comparaison de signaux dépassent la valeur de seuil.

5. Dispositif selon la revendication 4, **caractérisé en ce que** les moyens (32) de comparaison de valeurs de seuil sont agencés en outre de telle sorte qu'ils délivrent un signal d'identification lorsqu'un coefficient de corrélation dépasse par le haut la valeur de seuil, pour une première partie du signal provenant de la première mémoire (22) et atteint ou tombe au-dessous de la valeur zéro pour une seconde partie du signal, qui est enregistrée dans le temps après la première partie de signal.

6. Dispositif selon la revendication 3, et l'une des revendications 4 et 5, **caractérisé par** des moyens de localisation (38) qui sont reliés aux moyens (32) de comparaison de valeurs de seuil et aux moyens de détection (30) et sont agencés de telle sorte qu'ils associent un signal de localisation à la partie du signal de mesure située dans la première mémoire (22), pour laquelle le signal formé par le coefficient de corrélation possède un maximum à l'intérieur de la partie du signal formé par les coefficients de corrélation, pour laquelle les moyens (32) de comparaison de valeur de seuil délivrent un signal d'identification.

7. Dispositif selon la revendication 6, **caractérisé par** des moyens (34) de formation de valeurs de seuil, qui sont reliés à la mémoire de valeur de seuil (36) aux moyens de localisation (38) et sont agencés de telle sorte qu'ils forment une nouvelle valeur de seuil après l'apparition d'un signal de localisation, de telle sorte que le coefficient de corrélation associé au signal de localisation intervient en étant pondéré dans la formation de la nouvelle valeur de seuil.

8. Dispositif selon l'une des revendications 1 à 7, **caractérisé par** les moyens de formation de signal de comparaison pour former un nouveau signal de comparaison, qui sont reliés à la seconde mémoire (26) et sont agencés de telle sorte qu'une partie mesurée du signal, qui correspond à la caractéristique du signal devant être détectée, est transformée en le signal de comparaison de telle sorte que son intégrale dans le temps est nulle et que le signal de comparaison ainsi formé est transmis dans la seconde mémoire (26).

9. Dispositif selon l'une des revendications 1 à 8, **caractérisé par** des moyens (40) d'adaptation du signal de comparaison pour adapter le signal de comparaison, qui sont reliés à la première mémoire (22), à la seconde mémoire (26) et aux moyens de localisation (38) et sont agencés de telle sorte qu'ils forment un nouveau signal de comparaison adapté lorsque les moyens de localisation (38) délivrent un signal de localisation, le signal de comparaison adapté étant formé moyennant l'utilisation de la partie du signal de mesure qui provient de la première mémoire (22) et à laquelle est associé le signal de localisation.

10. Dispositif selon la revendication 9, **caractérisé par** les moyens (40) d'adaptation du signal de comparaison, qui sont agencés de telle sorte que le signal de comparaison, valable avant l'adaptation, est pondéré par un facteur $1-\alpha$ pour la formation du signal de comparaison valable après l'adaptation, tandis que la partie du signal situé dans la première mémoire (22), qui a déclenché le signal d'identification à l'aide des moyens de comparaison de signaux et des moyens de localisation, intervient avec le facteur de pondération $\alpha$ dans le signal de comparaison valable après l'adaptation.

11. Dispositif selon l'une des revendications 8 à 10, **caractérisé en ce que** les moyens (40) de formation du signal de comparaison et/ou les moyens d'adaptation du signal de comparaison sont agencés de telle sorte que le signal de comparaison ainsi formé ou adapté est normalisé de telle sorte que son amplitude correspond à l'amplitude maximale du signal de mesure.

12. Dispositif selon l'une des revendications 1 à 11, **caractérisé par** une banque de données (42), qui contient plusieurs signaux de référence et est reliée à la seconde mémoire (26) de telle sorte que des signaux de référence peuvent être transmis de la banque de données dans la seconde mémoire (26) et inversement.

13. Dispositif selon l'une des revendications 1 à 12, **caractérisé en ce qu'**il comporte des moyens d'analyse pour analyser les éléments caractéristiques du signal de comparaison de préférence adapté.

14. Dispositif selon l'une des revendications 1 à 13, **caractérisé par** des moyens (10, 12) pour recevoir deux signaux cardiaques, dont l'un est associé au ventricule ou à l'oreillette gauche et dont l'autre est associé au ventricule ou à l'oreillette droite, des moyens reliés à ces moyens et servant à former un signal bimodal à partir de deux signaux cardiaques de telle sorte que le signal bimodal contient une caractéristique d'un premier signal avant son transfert dans l'autre ventricule ou oreillette respectif et la caractéristique correspondante après son transfert de sorte que la caractéristique est contenue dans un intervalle de temps correspondant au temps de transfert, une fois sous sa forme avant le transfert et une fois sous sa forme après ce transfert, dans le signal bimodal et par le fait que la seconde mémoire contient un signal de comparaison bimodal, qui peut être adapté au signal bimodal de telle sorte qu'après l'adaptation du signal de comparaison bimodal au signal bimodal, le temps de transfert peut être déterminé au moyen d'une analyse du signal de comparaison.

Fig. 1

$$g(z) := - \left( z \exp \left( \frac{-z^2}{2} \right) \right)$$

Fig. 2

$$g(z) := - \left[ (z^2 - 1) \cdot \exp \left( \frac{-z^2}{2} \right) \right]$$

Fig. 3

Start oder
Restart

Initialisiere wWav mit iWav; Initialisiere detThr mit iDetThr; Initialisiere bIndex mit der Position wLen

Initialisiere detState mit Null; Initialisiere maxccVal mit dem größtmöglichen negativen Wert; Initialisiere maxccLoc mit Null

Neue Meßsignalwerte bei bIndex? — Nein

Ja

Berechne den Korrelationskoeffizienten ccVal unter Verwendung von wWav und den wLen entsprechenden Meßsignalwerten vor bIndex.

ccVal > maxccVal? — Ja — Setze maxccVal = ccVal
Setze maxccLoc = Lokation des Meßsignalwertes

Nein

ccVal > detThr? — Ja — Wenn detState = 0, Setze detState = 1

Nein

ccVal < 0? — Ja — Wenn detState = 1, Setze detState = 2

Nein

Nein — detState = 2? — Ja

Berechne den Korrelationskoeffizienten ccVal unter Verwendung von wWav und den wLen entsprechenden Meßsignalwerten nach bIndex.

ccVal > maxccVal? — Ja — Setze maxccVal = ccVal
Setze maxccLoc = featureLoc

Nein

Nein — Fertig? — Ja — Speichere die wLen entsprechende Zahl von Signalmeßwerten beginnend bei featureLoc

Passe wWav an die Meßsignalwerte bei featureLoc an, berücksichtigt die Nullsummenbedingung und Norminierung auf die Meßsignaldynamik

Lokalisiere das nächste Merkmal

Passe detThr an denjenigen maxccVal an der dem mit featureLoc verknüpften Meßsignalabschnitt zugeordnet ist.

Im Falle einer Refraktärzeit: Setze bIndex auf eine Position die tRef / tSamp Speicherstellen vor featureLoc liegt.

Fig. 4

$$g(z) := \mathrm{if}\left(.75 \le |z| \le 1.25, \frac{1 + \cos(4 \cdot \pi \cdot z)}{2}, 0\right) - \mathrm{if}\left[-5 \le z \le 5, \left(\frac{1 + \cos\left(\frac{\pi \cdot z}{5}\right)}{20}\right), 0\right]$$

Fig. 5

Fig. 6a

Fig. 6b

Fig. 6c

Fig. 6d